(19) Europäisches Patentamt
European Patent Office
Office européen des brevets

(11) EP 1 828 184 B1

(12) **EUROPEAN PATENT SPECIFICATION**

(45) Date of publication and mention of the grant of the patent:
**16.09.2009 Bulletin 2009/38**

(21) Application number: **05826556.2**

(22) Date of filing: **01.12.2005**

(51) Int Cl.:
***C07D 471/04*** *(2006.01)* ***A61K 31/435*** *(2006.01)*
***A61P 35/00*** *(2006.01)* ***C07D 249/00*** *(2006.01)*
***C07D 221/00*** *(2006.01)*

(86) International application number:
**PCT/EP2005/012855**

(87) International publication number:
**WO 2006/058752 (08.06.2006 Gazette 2006/23)**

(54) **[1,2,4]TRIAZOLO[4,3-A]PYRIDINE DERIVATIVES FOR THE TREATMENT OF HYPERPROLIFERATIVE DISEASES**

[1,2,4]TRIAZOLO[4,3-A]PYRIDIN-DERIVATIVE ZUR BEHANDLUNG HYPERPROLIFERATIVER ERKRANKUNGEN

DÉRIVÉS DE [1,2,4]TRIAZOLO[4,3-A]PYRIDINE POUR LE TRAITEMENT DE MALADIES HYPERPROLIFÉRATIVES

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **01.12.2004 EP 04028439**

(43) Date of publication of application:
**05.09.2007 Bulletin 2007/36**

(73) Proprietor: **Merck Serono SA**
**1267 Coinsins, Vaud (CH)**

(72) Inventors:
- **ABEL, Ulrich**
**69126 Heidelberg (DE)**
- **DEPPE, Holger**
**4052 Basel (CH)**
- **FEURER, Achim**
**79424 Auggen (DE)**
- **GRÄDLER, Ulrich**
**69115 Heidelberg (DE)**
- **OTT, Inge**
**68775 Ketsch (DE)**
- **MATASSA, Victor Giulio**
**E-08198 Barcelona (ES)**

(74) Representative: **Grünecker, Kinkeldey, Stockmair & Schwanhäusser Anwaltssozietät**
**Leopoldstrasse 4**
**80802 München (DE)**

(56) References cited:
**WO-A-99/01421**
**WO-A-03/077855**
**WO-A-03/077914**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### *Field of the invention*

The invention relates to a series of substituted 7-arylamino[1,2,4]triazolo[4,3-a]pyridine derivatives that are useful in the treatment of hyperproliferative diseases; such as cancer and inflammation, in mammals. Also disclosed are these compounds for use in the treatment of hyperproliferative diseases in mammals, especially humans, and pharmaceutical compositions containing such compounds.

### *Summary of the related art*

The Ras/Raf/MEK/ERK pathway is a central signal transduction pathway, which transmits signals from multiple cell surface receptors to transcription factors in the nucleus, which regulate gene expression. This pathway is frequently referred to as the MAP kinase pathway as MAPK stands for mitogen-activated protein kinase indicating that this pathway can be stimulated by mitogens, cytokines and growth factors (Steelman et al., Leukemia 2004, 18, 189-218). Depending upon the stimulus and cell type, this pathway can transmit signals, which result in the prevention or induction of apoptosis or cell cycle progression. The Ras/Raf/MEK/ERK pathway has been shown to play important roles in cell proliferation and the prevention of apoptosis. Aberrant activation of this pathway is commonly observed in malignantly transformed cells. Amplification of ras proto-oncogenes and activating mutations that lead to the expression of constitutively active Ras proteins are observed in approximately 30% of all human cancers (Stirewalt et al., Blood 2001, 97, 3589-95). Mutated, oncogenic forms of Ras are found in 50% of colon and >90% pancreatic cancers as well as many other types of cancers (Kohl et al., Science 1993, 260, 1834-1837). The effects of Ras on proliferation and tumorigenesis have been documented in immortal cell lines (McCubrey et al., Int J Oncol 1995, 7, 295-310). *b*Raf mutations have been identified in more than 60% of malignant melanoma (Davies, H et al., Nature 2002, 417, 949-954). Given the high level of mutations that have been detected at Ras, this pathway has always been considered a key target for therapeutic intervention (Chang et al., Leukemia 2003, 17, 1263-93).

The Ras/Raf/MEK/ERK signaling pathway can exert proliferative or antiproliferative effects through downstream transcription factor targets including NF-κB, CREB, Ets-1, AP-1 and c-Myc. ERKs can directly phosphorylate Ets-1, AP-1 and c-Myc, which lead to their activation. Alternatively, ERKs can phosphorylate and activate a downstream kinase target RSK, which then phosphorylates and activates transcription factors, such as CREB. These transcription factors induce the expression of genes important for cell cycle progression, for example, Cdks, cyclins, growth factors, and apoptosis prevention, for example, antiapoptotic Bcl-2 and cytokines. Overall, treatment of cells with growth factors leads to the activation of ERKs which results in proliferation and, in some cases, differentiation (Lewis et al., Adv. Cancer Res, 1998, 74, 49-139).

MEK proteins are the primary downstream targets of Raf. The MEK family of genes consists of five genes: MEK1, MEK2, MEK3, MEK4 and MEK5. This family of dual-specificity kinases has both serine/threonine and tyrosine kinase activity. The structure of MEK consists of an amino-terminal negative regulatory domain and a carboxy-terminal MAP kinase-binding domain, which is necessary for binding and activation of ERKs. Deletion of the regulatory MEK1 domain results in constitutive MEK1 and ERK activation (Steelman et al., Leukemia 2004, 18, 189-218).

MEK1 is a 393-amino-acid protein with a molecular weight of 44 kDa (Crews et al., Science 1992, 258, 478-80). MEK1 is modestly expressed in embryonic development and is elevated in adult tissue with the highest levels detected in brain tissue. MEK1 requires phosphorylation of S218 and S222 for activation, and substitution of these residues with D or glutamic acid (E) led to an increase in activity and foci formation in NIH3T3 cells (Huang et al., Mol Biol Cell, 1995, 6, 237-45). Constitutive activity of MEK1 in primary cell culture promotes senescence and induces p53 and $p16^{INK4a}$, and the opposite was observed in immortalized cells or cells lacking either p53 or $p16^{INK4a}$ (Lin et al., Genes Dev, 1998, 12, 3008-3019). Constitutive activity of MEK1 inhibits NF- κB transcription by negatively regulating $p38^{MAPK}$ activity (Carter et al., J Biol Chem 2000, 275, 27858-64). The main physiological substrates of MEK are the members of the ERK (extracellular signal-regulated kinase) or MAPK (mitogen activated protein kinase) family of genes. Aberrant expression of MEK1 has been detected in many different types of cancer, and mutated forms of MEK1 will transform fibroblast, hematopoietic and other cell types.

Constitutive activation of MEK1 results in cellular transformation. It therefore represents a likely target for pharmacological intervention in proliferative and inflammatory diseases (Lee et al., Nature 1994, 372, 739-746; Dudley et al., Proc. Natl. Acad. Sci. U.S.A. 1995, 92, 7686-7689).

Useful inhibitors of MEK have been developed that show potential therapeutic benefit in several studies. For example, small molecule MEK inhibitors have been shown to inhibit human tumor growth in nude mouse xenografts (Yeh, T. et al, Proceedings of the American Association of Cancer Research 2004, 45, Abs 3889 and Lee, P. et al., Proceedings of the American Association of Cancer Research 2004, 45, Abs 3890). MEK inhibitors also entered clinical trials, i.e. ARRY142886 (Wallace, E. et al, Proceedings of the American Association of Cancer Research 2004, 45, Abs

3891), PD-0325901 (Swanton C, Johnston S IDDB MEETING REPORT 2003, February 13-1) and PD-184352 (Waterhouse et al., Proceedings of the American Society for Clinical Oncology 2003, 22, Abs 816).

Compounds suitable as MEK inhibitors are also disclosed in US 5,525,625; WO 98/43960; WO 99/01421; WO 99/01426; WO 00/41505; WO 00/42002; WO 00/42003; WO 00/41994; WO 00/42022; WO 00/42029; WO 00/68201; WO 01/68619; WO 02/06213; WO03/035626; A2; WO 03/077855; WO03/077914; WO2004/005284; WO2004/056789. Of these, WO 03/077855 and WO03/077914 relate to benzimidazole derivatives having a substituted phenyl group linked via an amino spacer to the benzimidiazole.

However, PD-184352 was lacking efficacy in clinical phase II trials. Tumors were much less responsive, as no partial responses and only a few patients with stable disease were observed. As a result, the clinical trials of this molecule were suspended (McInnes C *IDDB MEETING REPORT* 2003). PD-184352 was limited by poor solubility, high metabolic clearance and low bioavailability. This exemplifies the need for novel MEK inhibitors with superior pharmacological properties.

***Description of the invention***

In view of the foregoing it is the object of the present invention to provide novel MEK inhibitors useful in the treatment of hyperproliferative diseases related to the hyperactivity of MEK as well as diseases modulated by the MEK cascade, such as cancer and inflammation, in mammals with superior pharmacological properties both with respect to their activities as well as their solubility, metabolic clearance and bioavailability characteristics.

As a result, this invention provides novel, substituted 7-arylamino[1,2,4]triazolo[4,3-a]pyridine derivatives and pharmaceutically acceptable salts, solvates or prodrugs thereof, that are MEK inhibitors and useful in the treatment of the above mentioned diseases.

The compounds are defined by Formula (I):

Formula (I) 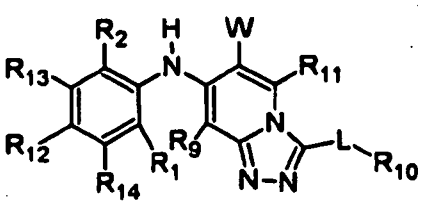

a pharmaceutically acceptable salt or solvate thereof, wherein:

$R_1$, $R_2$, $R_9$, $R_{11}$ $R_{12}$, $R_{13}$ and $R_{14}$ are independently selected from hydrogen, halogen, cyano, nitro, azido, $-OR_3$, $-C(O)R_3$, $-C(O)OR_3$, $-NR_4C(O)OR_6$, $-OC(O)R_3$, $-NR_4S(O)_jR_6$, $-S(O)_jNR_3R_4$, $-S(O)_jNR_4C(O)R_3$, $-C(O)NR_4S(O)_jR_6$, $S(O)_jR_6$, $-NR_4C(O)R_3$, $-C(O)NR_3R_4$, $-NR_5C(O)NR_3R_4$, $-NR_5C(NCN)NR_3R_4$, $-NR_3R_4$ and $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, $-S(O)_j(C_1$-$C_6$ alkyl), $-S(O)_j(CR_4R_5)_m$-aryl, aryl, arylalkyl, heteroaryl, heteroarylalkyl, heterocyclyl, heterocyclylalkyl, $-O(CR_4R_5)_m$-aryl, $-NR_4(CR_4R_5)_m$-aryl, $-O(CR_4R_5)_m$-heteroaryl, $-NR_4(CR_4R_5)_m$-heteroaryl, $-O(CR_4R_5)_m$-heterocyclyl, $-NR_4(CR_4R_5)_m$-heterocyclyl, and $-S(C_1$-$C_2$ alkyl) substituted with 1 to 5 F, where each alkyl, alkenyl, alkynyl, cycloalkyl, aryl, heteroaryl and heterocyclyl is unsubstituted or substituted as defined in the following;

$R_{10}$ is selected from hydrogen, $-OR_3$, $-C(O)R_3$,$-C(O)OR_3$, $-NR_4C(O)OR_6$, $-OC(O)R_3$, $-NR_4S(O)_jR_6$, $-S(O)_jNR_3R_4$, $-S(O)_jNR_4C(O)R_3$, $-C(O)NR_4S(O)_jR_6$, $S(O)_jR_6$, $-NR_4C(O)R_3$, $-C(O)NR_3R_4$, $-NR_5C(O)NR_3R_4$, $-NR_5C(NCN)NR_3R_4$, $-NR_3R_4$ ; $-S(O)_j(C_1$-$C_6$ alkyl), $-S(O)_j(CR_4R_5)_m$-aryl, $-O(CR_4R_5)_m$-aryl, $-NR_4(CR_4R_5)_m$-aryl, $-O(CR_4R_5)_m$-heteroaryl, $-NR_4(CR_4R_5)_m$-heteroaryl, $-O(CR_4R_5)_m$-heterocyclyl, $-NR_4(CR_4R_5)_m$-heterocyclyl, and $-S(C_1$-$C_2$ alkyl) substituted with 1 to 5 F, where each, aryl, heteroaryl and heterocyclyl is unsubstituted or substituted as defined in the following;

L is selected from a bond, $C_1$-$C_{10}$ alkyl, $C_2$-$C_{10}$ alkenyl, $C_2$-$C_{10}$ alkynyl, $C_3$-$C_{10}$ cy-

cloalkyl, $C_3$-$C_{10}$ cycloalkylalkyl, aryl, arylalkyl, heteroaryl, heteroarylalkyl, heterocyclyl, heterocyclylalkyl, where each alkyl, alkenyl, alkynyl, cycloalkyl, aryl, heteroaryl and heterocyclyl is unsubstituted or substituted as defined in the following;

$R_3$ is selected from hydrogen, trifluoromethyl, $C_1$-$C_{10}$ alkyl, $C_{2-10}$ alkenyl, $C_2$-$C_{10}$ alkynyl, $C_3$-$C_{10}$ cycloalkyl, $C_3$-$C_{10}$ cycloalkylalkyl, aryl, arylalkyl, heteroaryl, heteroarylalkyl, heterocyclyl, and heterocyclylalkyl, where each alkyl, alkenyl, alkynyl, cycloalkyl, aryl, heteroaryl and heterocyclyl is unsubstituted or substituted as defined in the following;

$R_4$ is selected from hydrogen or $C_1$-$C_6$ alkyl whereby alkyl may be unsubstituted or substituted as defined in the following; or

$R_3$ and $R_4$ can be taken together with the atom to which they are attached to form a 4 to 10 membered heteroaryl or heterocyclic ring, each of which is unsubstituted or substituted as defined in the following;

$R_5$ is selected from hydrogen or $C_1$-$C_6$ alkyl whereby alkyl may be unsubstituted or substituted as defined in the following; or

$R_4$ and $R_5$ can be taken together with the atom to which they are attached to form a 4 to 10 membered carbocyclic, heteroaryl or heterocyclic ring, each of which is unsubstituted or substituted as defined in the following;

$R_6$ is selected from trifluoromethyl, $C_1$-$C_{10}$ alkyl, $C_3$-$C_{10}$ cycloalkyl, aryl, arylalkyl, heteroaryl, heteroarylalkyl, heterocyclyl, and heterocyclylalkyl, where each alkyl, cycloalkyl, aryl, heteroaryl and heterocyclyl unsubstituted or substituted as defined in the following;

W is selected from heteroaryl containing 1-4 heteroatoms or heterocyclyl containing 1-4 heteroatoms each of which is unsubstituted or substituted by 1 to 5 substituents $ZR_{15}$; or W is $-C(O)OR_{15}$, $-C(O)NR_4R_{15}$, $-C(O)NR_4OR_{15}$, $-C(O)(C_3$-$C_{10}$ cycloalkyl), $-C(O)(C_2$-$C_{10}$ alkyl), -C(O)(aryl), -C(O)(heteroaryl), -C(O)(heterocyclyl), $-S(O)_jNR_4R_{15}$, $-S(O)_jNR_4OR_{15}$, $-S(O)_jNR_4C(O)R_{15}$, or $-C(O)NR_4S(O)_jR_6$, whereby $R_4$ and $R_{15}$ are as defined herein or may form together a 3 to 7 membered ring with 1 or 2 N atoms and optionally an O atom,

Z is a bond, $NR_{16}$, O, $NR_{16}SO_2$ or S,

$R_{15}$ is independently selected from hydrogen, trifluoromethyl, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, aryl, arylalkyl, heteroaryl, heteroarylalkyl, heterocyclyl, and heterocyclylalkyl, where each alkyl, alkenyl, alkynyl, cycloalkyl, aryl, heteroaryl and heterocyclyl is unsubstituted or substituted as defined in the following;

$R_{16}$ is selected from hydrogen or $C_1$-$C_{10}$ alkyl, or $R_{15}$ and $R_{16}$ form together a 4 to 10 membered cyclic ring with 1 or 2 N atoms and optionally an O atom, said ring being unsubstituted or substituted as defined in the following;

m is 0, 1, 2, 3, 4 or 5 ;and

j is 1 or 2.

In a preferred embodiment, the variants $R_1$-$R_{16}$, L, W and Z are defined as above on pages 4 to 5 but with the proviso that the compounds with the following formula including resolved enantiomers, diastereomers, solvates and pharmaceutically acceptable salts thereof are excluded:

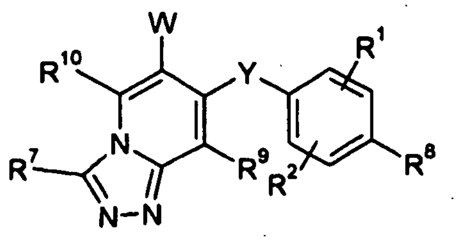

wherein

| | |
|---|---|
| Y | is NH; |
| $R^1$, $R^2$, $R^7$, $R^8$, $R^9$ $R^{10}$ | and are independently hydrogen, halogen, cyano, nitro, azido,$-OR^3$, $-C(O)R^3$, $-C(O)OR^3$, $-NR^4C(O)OR^6$, $-OC(O)R^3$, $-NR^4SO_2R^6$, $-SO_2NR^3R^4$, $-NR^4C(O)R^3$, $-C(O)NR^3R^4$, $-NR^5C(O)NR^3R^4$, $-NR^5C(NCN)NR^3R^4$, $-NR^3R^4$, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, $-S(O)_j(C_1$-$C_6$ alkyl), $-S(O)_j(CR^4R^5)_m$-aryl, aryl, arylalkyl, heteroaryl, heteroarylalkyl, heterocyclyl, heterocyclylalkyl, $-O(CR^4R^5)_m$-aryl, $-NR^5(CR^4R^5)_m$-aryl, $-O(CR^4R^5)_m$-heteroaryl, $-NR^4(CR^4R^s)_m$-heteroaryl, $-O(CR^4R^5)_m$-heterocyclyl or - $NR^4(CR^4R^5)_m$-heterocyclyl, wherein any of said alkyl, alkenyl, alkynyl, cycloalkyl, aryl, arylalkyl, heteroaryl, heteroarylalkyl, heterocyclyl and heterocyclylalkyl portions are optionally substituted with one or more groups independently selected from oxo, halogen, cyano, nitro, trifluoromethyl, difluoromethoxy, trifluoromethoxy, azido, $-NR^4SO_2R^6$, $-SO_2NR^3R^4$, $-C(O)R^3$, $-C(O)OR^3$, $-OC(O)R^3$, $-NR^4C(O)OR^6$, $-NR^4C(O)R^3$, $-C(O)NR^3R^4$, $-NR^3R^4$, $-NR^5C(O)NR^3R^4$, $-NR^5C(NCN)R^3R^4$, $-OR^3$, aryl, heteroaryl, arylalkyl, heteroarylalkyl, heterocyclyl, and heterocyclylalkyl, |
| $R^3$ | is hydrogen, trifluoromethyl, $C_1$-$C_{10}$ alkyl, $C_2$-$C_{10}$ alkenyl, $C_2$-$C_{10}$ alkynyl, $C_3$-$C_{10}$-cycloalkyl, $C_3$-$C_{10}$ cycloalkylalkyl, aryl, arylalkyl, heteroaryl, heteroarylalkyl, heterocyclyl, heterocyclylalkyl, wherein any of said alkyl, alkenyl, alkynyl, cycloalkyl, aryl, arylalkyl, heteroaryl, heteroarylalkyl, heterocyclyl and heterocyclylalkyl portions are optionally substituted with one or more groups independently selected from oxo, halogen, cyano, nitro, trifluoromethyl, difluoromethoxy, trifluoromethoxy, azido, -NR'$SO_2$R'''', $-SO_2$NR'R'', -C(O)R', -C(O)OR', -OC(O)R', -NR'C(O)OR'''', -NR'C(O)R'', -C(O)NR'R'', -SR', -S(O)R'''', $-SO_2$R'''', -NR'R'', -NR'C(O)NR''R''', -NR'C(NCN)N''R''', -OR', aryl, heteroaryl, arylalkyl, heteroarylalkyl, heterocyclyl, and heterocyclylalkyl, or |
| $R^3$ and $R^4$ | together with the atom to which they are attached form a 4 to 10 membered heteroaryl or heterocyclic ring, wherein any of said heteroaryl or heterocyclic rings are optionally substituted with one or more groups independently selected from halogen, cyano, nitro, trifluoromethyl, difluoromethoxy, trifluoromethoxy, azido, NR'$SO_2$R'''', $-SO_2$NR'R'', -C(O)R', -C(O)OR', -OC(O)R', -NRC(O)OR'', -NR'C(O)R'', -C(O)NRR'' $-SO_2$R'''', -NR'R'', -NRC(O)NR'R''', -NR'C(NCN)NR''R''', -OR', aryl, heteroaryl, arylalkyl, heteroarylalkyl, heterocyclyl, and heterocyclylalkyl; |
| R', R'' and R''' | independently are hydrogen, lower alkyl, lower alkenyl, aryl and arylalkyl, and |
| R'''' | is lower alkyl, lower alkenyl, aryl or arylalkyl, or any two of R', R'', R''' or R'''' together with the atom to which they are attached form a 4 to 10 membered carbocyclic, heteroaryl or heterocyclic ring, wherein any of said alkyl, alkenyl, aryl, arylalkyl carbocyclic rings, heteroaryl rings or heterocyclic rings are optionally substituted with one or more groups independently selected from halo, cyano, nitro, trifluoromethyl, difluoromethoxy, trifluoromethoxy, azido, aryl, heteroaryl, arylalkyl, heteroarylalkyl, heterocyclyl, and heterocyclylalkyl; |
| $R^4$ and $R^5$ | independently are hydrogen or $C_1$-$C_6$ alkyl, or |
| $R^4$ and $R^5$ | together with the atom to which they are attached form a 4 to 10-membered carbocyclic, heteroaryl or heterocyclic ring, wherein said alkyl or any of said carbocyclic, heteroaryl and |

heterocyclic rings are optionally substituted with one or more groups independently selected from cyano, halogen, nitro, trifluoromethyl, difluoromethoxy, trifluoromethoxy, azido, -NR'$SO_2$R'''', -$SO_2$NR'R'', -C(O)R'''', C(O)OR', -OC(O)R', -NR'C(O)OR'''', -NR'C(O)R'', -C(O)NR'R'', -$SO_2$R'''', -NR'R'', -NRC(O)NR''R''', -NR'C(NCN)NR''R''', -OR', aryl, heteroaryl, arylalkyl, heteroarylalkyl, heterocyclyl, and heterocyclylalkyl;

$R^6$ is trifluoromethyl, $C_1$-$C_{10}$ alkyl, $C_3$-$C_{10}$ cycloalkyl, aryl, arylalkyl, heteroaryl, heteroarylalkyl, heterocyclyl or heterocyclylalkyl, wherein any of said alkyl, cycloalkyl, aryl, arylalkyl, heteroaryl, heteroarylalkyl, heterocyclyl and heterocyclylalkyl portions are optionally substituted with one or more groups independently selected from oxo, halogen, cyano, nitro, trifluoromethyl, difluoromethoxy, trifluoromethoxy, azido, -NR'$SO_2$R'''', -$SO_2$NR'R'', -C(O)R', C(O)OR', -OC(O)R', -NR'C(O)OR'''', -NR'C(O)R'', -C(O)NR'R'', -$SO_2$R'''', -NR'R', -NR'C(O)NR''R''', -NR'C(NCN)NR''R''', -OR', aryl, heteroaryl, arylalkyl, heteroarylalkyl, heterocyclyl, and heterocyclylalkyl;

W is heteroaryl, heterocyclyl, -C(O)$OR^3$, -C(O)$NR^3R^4$, -C(O)$NR^4OR^3$, -C(O)($C_3$-$C_{10}$ cycloalkyl), -C(O)($C_1$-$C_{10}$ alkyl), -C(O)(aryl), -C(O)(heteroaryl), -C(O)(heterocyclyl), -C(O)NH($SO_2$)$CH_3$, wherein any of said heteroaryl, heterocyclyl, -C(O)$OR^3$, -C(O)$NR^3R^4$, -C(O)$NR^4OR^3$, -C(O)($C_3$-$C_{10}$ cycloalkyl),- C(O)($C_1$-$C_{10}$ alkyl), -C(O)(aryl), -C(O)(heteroaryl), -C(O)(heterocyclyl), C(O)NH($SO_2$)$CH_3$ are optionally substituted with one or more groups independently selected from -$NR^3R^4$, -$OR^3$, $R^2$, $C_1$-$C_{10}$ alkyl, $C_2$-$C_{10}$ alkenyl, and $C_2$-$C_{10}$ alkynyl, wherein any of said $C_1$-$C_{10}$ alkyl, $C_2$-$C_{10}$ alkenyl, and $C_2$-$C_{10}$ alkynyl, are optionally substituted with 1 or more groups independently selected from -$NR^3R^4$ and - $OR^3$;

m is 0, 1, 2, 3 ,4 or 5; and

j is 1 or 2.

In preferred embodiments, the variants have the following meanings:

$R_1$ is as defined above, preferably hydrogen, halo, $C_1$-$C_4$ alkyl, $C_3$-$C_4$ cycloalkyl, $C_2$-$C_4$ alkenyl, $C_2$-$C_4$ alkynyl, cyano, nitro, $OR_3$ or $NR_3R_4$; more preferably hydrogen, halo or $C_1$-$C_4$ alkyl, still more preferably hydrogen or halo, most preferably hydrogen or F. In one embodiment, $R_1$ is hydrogen.

In a further embodiment, $R_1$ is -$S(O)_jNR_4C(O)R_3$, -$C(O)NR_4S(O)_jR_6$, or $S(O)_jR_6$.

$R_2$ is as defined above, preferably hydrogen, halo, $C_1$-$C_4$ alkyl, $C_3$-$C_4$ cycloalkyl, $C_2$-$C_4$ alkenyl, $C_2$-$C_4$ alkynyl, cyano, nitro, $OR_3$ or $NR_3R_4$; more preferably hydrogen, halo or $C_1$-$C_2$ alkyl, still more preferably halo or methyl, most preferably Cl, F or methyl. In one embodiment, $R_2$ is methyl. In another embodiment, methyl is preferably further substituted by 1, 2 or 3 fluorines, preferably 3 fluorines. Most preferably, $R_2$ is F.

In still another embodiment, $R_2$ is -$S(O)_jNR_4C(O)R_3$, -$C(O)NR_4S(O)_jR_6$, or $S(O)_jR_6$.

$R_9$ is as defined above, preferably hydrogen, halo, $C_1$-$C_4$ alkyl, $C_3$-$C_4$ cycloalkyl, $C_2$-$C_4$ alkenyl, $C_2$-$C_4$ alkynyl, cyano, nitro, $OR_3$ or $NR_3R_4$; more preferably hydrogen, halo or $C_1$-$C_4$ alkyl, still more preferably hydrogen, methyl or halo, most preferably hydrogen, methyl, Cl or F. In one embodiment, $R_9$ is hydrogen.

In another embodiment, $R_9$ is -$S(O)_jNR_4C(O)R_3$, -$C(O)NR_4S(O)_jR_6$, or $S(O)_jR_6$.

$R_{10}$ is as defined above, preferably hydrogen, -$OR_3$, -$C(O)R_3$, -$C(O)OR_3$, -$NR_4C(O)OR_6$, -$OC(O)R_3$, -$NR_4S(O)_2R_6$, -$S(O)_2NR_3R_4$, $S(O)_2R_6$, -$NR_4C(O)R_3$, -$C(O)NR_3R_4$, -$NR_5C(O)NR_3R_4$, -$NR_3R_4$, more preferably hydrogen, -$OR_3$, -$NR_4C(O)R_3$, -$C(O)NR_3R_4$, -$NR_3R_4$, still more preferably hydrogen, -$OR_3$, -$NR_3R_4$, most preferably hydrogen. In preferred embodiments $R_3$ and $R_4$ are independently $C_1$-$C_6$ alkyl, more preferably $C_1$-$C_4$ alkyl, optionally substituted by 1 or 2 alkyl amino, dialkyl amino, amino, O-alkyl, hydroxy, or $R_3$ and $R_4$ form together a cyclic ring with 1 or 2 N atoms and optionally an O atom, said ring being optionally substituted by 1 or 2 alkyl amino, amino, hydroxy or O-alkyl.

In a further embodiment, $R_{10}$ is -$S(O)_jNR_4C(O)R_3$, -$C(O)NR_4S(O)_jR_6$, -$S(O)_jR_6$, -$S(O)_j$($C_1$-$C_6$ alkyl), -$S(O)_j(CR_4R_5)_m$-aryl, -$O(CR_4R_5)_m$-aryl, -$NR_4(CR_4R_5)_m$-aryl, -$O(CR_4R_5)_m$-heteroaryl, -$NR_4(CR_4R_5)_m$-heteroaryl, -$O(CR_4R_5)_m$-heterocyclyl, or -$NR_4(CR_4R_5)_m$-heterocyclyl and -S($C_1$-$C_2$alkyl) substituted with 1 to 5 F, where each aryl, heteroaryl and heterocyclyl is substituted or unsubstituted.

L is as defined above, preferably a bond, $C_1$-$C_6$ alkyl, $C_2$-$C_6$ alkenyl, $C_2$-$C_6$ alkynyl, $C_3$-$C_6$ cycloalkyl, $C_3$-$C_{10}$ cycloalkylalkyl, arylalkyl, heteroarylalkyl, heterocyclyl, heterocyclylalkyl, more preferably a bond, $C_1$-$C_5$ alkyl, most preferably a bond, methylene, ethylene, n-propylene or n-butylene. In one embodiment, L is ethylene, n-propylene or n-butylene. In another embodiment, L is a bond or methylene. In the definition of L all moieties are divalent so that L serves as a linker between the nitrogen atom and $R_{10}$.

$R_{11}$ is as defined above, preferably hydrogen, halo, $C_1$-$C_4$ alkyl, $C_3$-$C_4$ cycloalkyl, $C_2$-$C_4$ alkenyl, $C_2$-$C_4$ alkynyl, cyano, nitro, $OR_3$ or $NR_3R_4$; more preferably hydrogen, halo or $C_1$-$C_4$ alkyl or O-$C_1$-$C_4$ alkyl, still more preferably

hydrogen, methyl, O-methyl or halo, most preferably hydrogen, methyl, Cl, Br or F. In one embodiment, $R_{11}$ is hydrogen. In another embodiment, $R_{11}$ is methyl. In yet another embodiment, methyl is preferably further substituted by 1, 2 or 3 fluorines, preferably 3 fluorines.

In another embodiment, $R_{11}$ is $-S(O)_jNR_4C(O)R_3$, $-C(O)NR_4S(O)_jR_6$, or $S(O)_jR_6$. $R_{12}$ is as defined above, preferably hydrogen, halo, $C_1$-$C_{10}$ alkyl, $C_3$-$C_{10}$ cycloalkyl, $C_2$-$C_{10}$ alkenyl, $C_2$-$C_{10}$ alkynyl, cyano, nitro, azido; $NR_4SO_2R_6$; $SO_2NR_3R_4$; $SO_2R_6$; $C(O)NR_3R_4$; $C(O)OR_3$; $OR_3$, $NR_3R_4$ or $-S(C_1$-$C_2$ alkyl) substituted with 1 to 5 F, more preferably hydrogen, halo, nitro, $C_1$-$C_4$ alkyl, O-$C_1$-$C_4$ alkyl, $SCF_3$, $SCHF_2$, $SCH_2F$, $SO_2NR_3R_4$ or $C(O)NR_3R_4$, still more preferably hydrogen, F, Cl, Br, I, nitro, methyl, ethyl, n-propyl, i-propyl, cyclopropyl, O-methyl, $SCF_3$, $SCHF_2$, $SCH_2F$, $SO_2NR_3R_4$ or $C(O)NR_3R_4$, most preferably hydrogen I, Cl, Br, $SCF_3$, $SCHF_2$, $SCH_2F$, methyl or O-methyl. In one embodiment $R_{12}$ is hydrogen. In another embodiment, $R_{12}$ is methyl, $SCF_3$, $SCHF_2$, $SCH_2F$ or O-methyl, wherein methyl or O-methyl is preferably unsubstituted or further substituted by 1, 2 or 3 fluorines, preferably 2 or 3 fluorines. In preferred embodiments of $R_{12}$, $R_3$ and $R_4$ are independently $C_1$-$C_6$ alkyl, more preferably $C_1$-$C_4$ alkyl, optionally substituted by 1 or 2 alkyl amino, dialkyl amino, amino, O-alkyl, hydroxy, or $R_3$ and $R_4$ form together a cyclic ring with 1 or 2 N atoms and optionally an O atom, said ring being optionally substituted by 1 or 2 alkyl amino, amino, hydroxy or O-alkyl. Most preferably, $R_{12}$ is Br or I.

In another embodiment, $R_{12}$ is $-S(O)_jNR_4C(O)R_3$, $-C(O)NR_4S(O)_jR_6$, or $S(O)_jR_6$.

$R_{13}$ is as defined above, preferably hydrogen, halo, $C_1$-$C_4$ alkyl, $C_3$-$C_4$ cycloalkyl, $C_2$-$C_4$ alkenyl or $C_2$-$C_4$ alkynyl, more preferably hydrogen, F, Cl or methyl, most preferably hydrogen or F. in one embodiment, $R_{13}$ is hydrogen.

In another embodiment, $R_{13}$ is $-S(O)_jNR_4C(O)R_3$, $-C(O)NR_4S(O)_jR_6$, or $S(O)_jR_6$.

$R_{14}$ is as defined above, preferably hydrogen, halo, $C_1$-$C_4$ alkyl, $C_3$-$C_4$ cycloalkyl, $C_2$-$C_4$ alkenyl or $C_2$-$C_4$ alkynyl, more preferably hydrogen, F, Cl or methyl, most preferably hydrogen or F. In one embodiment, $R_{14}$ is hydrogen.

In a further embodiment, $R_{14}$ is $-S(O)_jNR_4C(O)R_3$, $-C(O)NR_4S(O)_jR_6$, or $S(O)_jR_6$.

In a particular preferred embodiment, at least one of $R_1$, $R_2$, $R_9$, $R_{11}$, $R_{12}$, $R_{13}$ and $R_{14}$ is selected from $-S(O)_jNR_4C(O)R_3$, $-C(O)NR_4S(O)_jR_6$, and $S(O)_jR_6$.

As set forth above, the variants of each of $R_1$, $R_2$, $R_9$ to $R_{14}$ and L may be substituted. In this case they can be substituted with 1 to 5, preferably 1 to 3, more preferably 1 or 2 groups independently selected from oxo, halogen, cyano, nitro, $CF_3$, $CHF_2$, $CH_2F$, $OCF_3$, $OCHF_2$, $OCH_2F$, $SCF_3$, $SCHF_2$, $SCH_2F$, azido, $NR_4SO_2R_6$, $SO_2NR_3R_4$, $C(O)R_3$, $C(O)OR_3$, $OC(O)R_3$, $NR_4C(O)OR_6$, $NR_4C(O)R_3$, $C(O)NR_3R_4$, $NR_3R_4$, $NR_5C(O)NR_3R_4$, $NR_5C(NCN)NR_3R_4$, $OR_3$, aryl, heteroaryl, arylalkyl, heteroarylalkyl, heterocyclyl, and heterocyclylalkyl, preferably oxo, halogen, cyano, nitro, $CF_3$, $CHF_2$, $CH_2F$, $OCF_3$, $OCHF_2$, $OCH_2F$, $SCF_3$, $SCHF_2$, $SCH_2F$, azido, $NR_4SO_2R_6$, $SO_2NR_3R_4$, $C(O)R_3$, $C(O)OR_3$, $OC(O)R_3$, $OR_3$, more preferably oxo, halogen, cyano, nitro, trifluoromethyl, difluoromethoxy, trifluoromethoxy or azido, most preferably halogen, cyano, nitro, $CF_3$, $CHF_2$, $CH_2F$, $OCF_3$, $OCHF_2$, $OCH_2F$, $SCF_3$, $SCHF_2$, $SCH_2F$, OH, O-methyl, $NH_2$ or $N(methyl)_2$. When it is described that alkyl, alkenyl, alkynyl, cycloalkyl, aryl, heteroaryl and heterocyclyl are substituted, this refers to any alkyl, alkenyl, alkynyl, cycloalkyl, aryl, heteroaryl and heterocyclyl as a group or sub-structure such as in cycloalkylalkyl, arylalkyl, heteroarylalkyl, heterocyclylalkyl.

$R_3$ is as defined above, preferably hydrogen, trifluoromethyl, $C_1$-$C_4$ alkyl, $C_2$-$C_4$ alkenyl, $C_2$-$C_4$ alkynyl, $C_3$-$C_6$ cycloalkyl, $C_3$-$C_6$ cycloalkylalkyl, more preferably hydrogen or $C_1$-$C_4$ alkyl most preferably hydrogen, methyl or ethyl.

$R_4$ is as defined above, preferably hydrogen or $C_1$-$C_4$ alkyl, more preferably hydrogen, methyl or ethyl.

In one preferred embodiment, $R_3$ and $R_4$ can be taken together with the atom to which they are attached to form a 4 to 7, preferably 5 or 6, membered heteroaryl or heterocyclic ring.

$R_5$ is as defined above, preferably hydrogen or $C_1$-$C_4$ alkyl, more preferably hydrogen, methyl or ethyl.

In one embodiment, $R_4$ and $R_5$ can be taken together with the atom to which they are attached to form a 4 to 7, preferably 5 or 6, membered carbocyclic, heteroaryl or heterocyclic ring.

$R_6$ is as defined above, preferably trifluoromethyl, $C_1$-$C_4$ alkyl, $C_2$-$C_4$ alkenyl, $C_2$-$C_4$ alkynyl, $C_3$-$C_6$ cycloalkyl, $C_3$-$C_6$ cycloalkylalkyl, more preferably $C_1$-$C_4$ alkyl, most preferably methyl or ethyl.

As set forth above, the variants of each of $R_3$, $R_4$, $R_5$, $R_6$ or the rings formed by $R_3$ and $R_4$ and $R_4$ and $R_5$ may be substituted. In this case they can be substituted with 1 to 5, preferably 1 to 3, more preferably 1 or 2 groups independently selected from oxo, halogen, cyano, nitro, $CF_3$, $CHF_2$, $CH_2F$, $OCF_3$, $OCHF_2$, $OCH_2F$, azido, NR'$SO_2$R'''', $SO_2$NR'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''', OR', aryl, heteroaryl, arylalkyl, heteroarylalkyl, heterocyclyl, and heterocyclylalkyl, preferably oxo, halogen, cyano, nitro, $CF_3$, $CHF_2$, $CH_2F$, $OCF_3$, $OCHF_2$, $OCH_2F$, azido, NR'$SO_2$R'''', $SO_2$NR'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''' or OR', more preferably oxo, halogen, cyano, nitro, $CF_3$, $CHF_2$, $CH_2F$, $OCF_3$, $OCHF_2$, $OCH_2F$, azido, SR'''', S(O)R'''', $SO_2$R', NR'R'' or OR'. In one embodiment, $R_3$ is preferably oxo, halogen, nitro, trifluoromethyl, OH, O-methyl, $NH_2$ or $N(methyl)_2$. When it is described that alkyl, alkenyl, alkynyl, cycloalkyl, aryl, heteroaryl and heterocyclyl are substituted, this refers to any alkyl, alkenyl, alkynyl, cycloalkyl, aryl, heteroaryl and heterocyclyl as a group or sub-structure such as in cycloalkylalkyl, arylalkyl, heteroarylalkyl, heterocyclylalkyl.

R' is selected from hydrogen, $C_1$-$C_4$ alkyl, $C_2$-$C_4$ alkenyl, aryl and arylalkyl, preferably hydrogen or $C_1$-$C_4$ alkyl, more preferably hydrogen or methyl.

R" is selected from hydrogen, $C_1$-$C_4$ alkyl, $C_2$-$C_4$ alkenyl, aryl and arylalkyl, preferably hydrogen or $C_1$-$C_4$ alkyl, more preferably hydrogen or methyl.

R'" is selected from hydrogen, $C_1$-$C_4$ alkyl, $C_2$-$C_4$ alkenyl, aryl and arylalkyl, preferably hydrogen or $C_1$-$C_4$ alkyl, more preferably hydrogen or methyl.

R"" is selected from $C_1$-$C_4$ alkyl, $C_1$-$C_4$ alkenyl, aryl and arylalkyl, preferably $C_1$-$C_4$ alkyl, more preferably methyl.

Alternatively, any two of R', R", R"' or R"" can be taken together with the atom to which they are attached to form a 4 to 10 membered carbocyclic, heteroaryl or heterocyclic ring, each of which is optionally substituted with one to three groups independently selected from halogen, cyano; nitro, $CF_3$, $CHF_2$, $CH_2F$, $OCF_3$, $OCHF_2$, $OCH_2F$, azido, aryl, heteroaryl, arylalkyl, heteroarylalkyl, heterocyclyl, and heterocyclylalkyl, preferably halogen, cyano; nitro, trifluoromethyl, difluoromethoxy, trifluoromethoxy and azido.

W is as defined above, preferably heteroaryl containing 1, 2 or 3 heteroatoms, or heterocyclyl containing 1, 2,or 3 heteroatoms, more preferably heteroaryl, each of which is unsubstituted or substituted by 1 to 5, preferably 1 to 3, more preferably 1, substituents $ZR_{15}$, or W is $-C(O)OR_{15}$, $-C(O)NR_4R_{15}$, $-C(O)NR_4OR_{15}$, $-C(O)(C_3$-$C_{10}$ cycloalkyl), $-C(O)(C_2$-$C_{10}$ alkyl), $-S(O)_jNR_4C(O)R_{15}$, $-C(O)NR_4S(O)_jR_6$, $S(O)_jNR_4R_{15}$ or $S(O)_jNR_4OR_{15}$, more preferably W is heteroaryl containing 1, 2, or 3, specifically 2 or 3 N atoms, $C(O)NR_4OR_{15}$ or $S(O)_2NR_4OR_{15}$. $R_4$ and $R_{15}$ are as defined herein or may form together a 3 to 7 membered ring with 1 or 2 N atoms and optionally an O atom. When W is heteroaryl, it is preferably

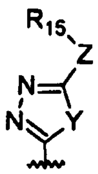

where Z and $R_{15}$ are as defined above, preferably Z is a bond, $NR_{16}$, $NR_{16}SO_2$ or O, more preferably $NR_{16}$, wherein $R_{16}$ is as defined above, preferably hydrogen or $C_1$-$C_4$ alkyl, more preferably hydrogen. $R_{15}$ is preferably selected from hydrogen, $C_1$-$C_4$ alkyl, $C_1$-$C_4$ alkenyl, $C_4$-$C_8$ cycloalkylalkyl, each may contain 1 N atom optionally an O atom, where alkyl, alkenyl or cycloalkylalkyl may be further substituted by 1 or 2 of OH, O-$C_1$-$C_4$ alkyl or NR'R", where R' and R" are independently hydrogen or $C_1$-$C_4$ alkyl where R' and R" may form a 3 to 7 membered ring with 1 or 2 N atoms and optionally an O atom. Alternatively, $R_{16}$ and $R_{15}$ may form together a 4 to 10 membered cyclic ring with 1 or 2 N atoms and optionally an O atom, said ring being optionally substituted by 1 or 2 alkyl amino, amino, hydroxy or O-alkyl. More preferably $R_{15}$ is $C_1$-$C_4$ alkyl or $C_1$-$C_4$ alkenyl optionally substituted with 1 substituent OH, O-Me, $NH_2$, NHmethyl, NHethyl, $N(methyl)_2$ or $N(ethyl)_2$.

Y is O or NR', preferably O.

Alternatively, W is preferably $-C(O)OR_{15}$, $-C(O)NR_4R_{15}$, $-C(O)NR_4OR_{15}$, $S(O)_jNR_4R_{15}$ or $S(O)_jNR_4OR_{15}$, more preferably $-C(O)NR_4OR_{15}$ or $S(O)_2NR_4OR_{15}$. In these cases $R_{15}$ is preferably as defined below.

In yet another preferred embodiment, W is $-C(O)NR_4S(O)_jR_6$, or $-S(O)_jNR_4C(O)R_{15}$, whereby $R_4$ and $R_{15}$ are as defined herein or may form together a 3 to 7 membered ring with 1 or 2 N atoms and optionally an O atom.

Z is as defined above, preferably a bond, $NR_{16}$, $NR_{16}SO_2$ or O, more preferably $NR_{16}$.

In another embodiment, Z is S.

$R_{15}$ is as defined above, preferably hydrogen, $C_1$-$C_4$ alkyl, $C_1$-$C_4$ alkenyl, $C_4$-$C_6$ cycloalkylalkyl, more preferably $C_1$-$C_4$ alkyl or $C_1$-$C_4$ alkenyl, yet more preferably $C_1$-$C_4$ alkyl. Alkyl, alkenyl cycloalkyl, alkynyl, aryl, heteroaryl or heterocyclyl may be further substituted with 1 to 5, preferably 1, 2 or 3, more preferably 1 or 2, substituents selected from $OR_3$ or NR'R" wherein $R_3$ is selected from hydrogen, $C_1$-$C_4$ alkyl or $C_1$-$C_4$ alkenyl, $C_4$-$C_6$ cycloalkylalkyl, more preferably hydrogen, methyl or ethyl, and where R' and R" are independently hydrogen or $C_1$-$C_4$ alkyl, or R' and R" may form a 3 to 7 membered ring with 1 or 2 N atoms and optionally an O atom, more preferably R' and R" are independently hydrogen, methyl or ethyl, still more preferably both R' and R" are methyl. Yet more preferably, $R_{15}$ may be substituted by 1 or 2 of OH, O-$C_1$-$C_4$ alkyl or NR'R".

Most preferably, $R_{15}$ is $C_1$-$C_4$ alkyl or $C_1$-$C_4$ alkenyl optionally substituted with 1 substituent OH, O-Me, $NH_2$, $N(methyl)_2$ or $N(ethyl)_2$.

Regarding $R_{15}$, when it is described that alkyl, alkenyl, alkynyl, cycloalkyl, aryl, heteroaryl and heterocyclyl are substituted, this refers to any alkyl, alkenyl, alkynyl, cycloalkyl, aryl, heteroaryl and heterocyclyl as a group or substructure such as in cycloalkylalkyl, arylalkyl, heteroarylalkyl, heterocyclylalkyl.

$R_{16}$ is as defined above, preferably hydrogen or $C_1$-$C_4$ alkyl, more preferably hydrogen.

Alternatively, $R_{16}$ and $R_{15}$ may form together a 4 to 10, preferably 5 to 6, membered cyclic ring with 1 or 2 N atoms and optionally an O atom, said ring being optionally substituted by 1 or 2 alkyl amino, amino, hydroxy or O-alkyl.

m is as defined above, preferably 0, 1, 2 or 3, more preferably 0,1 or 2, most preferably 1.

j is as defined above, preferably 2.

In the above, any of the preferred definitions for each variant can be combined with the preferred definition of the other variants.

The combinations as set forth in the claims are particularly preferred.

In the above and the following, the employed terms have independently the meaning as described below:

Aryl is an aromatic mono- or polycyclic moiety with preferably 6 to 20 carbon atoms which is preferably selected from phenyl, biphenyl, naphthyl, tetrahydronaphthyl, fluorenyl, indenyl or phenanthrenyl, more preferably phenyl or naphthyl.

Heteroaryl is an aromatic moiety having 6 to 20 carbon atoms with at least one ring containing a heteroatom selected from O, N and/or S, or heteroaryl is an aromatic ring containing at least one heteroatom selected from O, N and/or S and 1 to 6 carbon atoms. Preferably, heteroaryl contains 1 to 4, more preferably 1, 2 or 3 heteroatoms selected from O and/or N and is preferably selected from pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, triazolyl, thiadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. Spiro moieties are also included within the scope of this definition. Preferred heteroaryl include pyridinyl, imidazolyl, pyrimidinyl, pyrazolyl, triazolyl, pyrazinyl, tetrazolyl, isoxazolyl, oxazolyl, isothiazolyl, oxadiazolyl, triazolyl. Heteroaryl groups are optionally mono-, di-, or trisubstituted with, e.g., halogen, lower alkyl, lower alkoxy, haloalkyl, aryl, heteroaryl, and hydroxy.

Heterocyclyl is a saturated or unsaturated ring containing at least one heteroatom selected from O, N and/or S and 1 to 6 carbon atoms. Preferably, heterocyclyl contains 1 to 4, more preferably 1, 2 or 3 heteroatoms selected from O and/or N and is preferably selected from pyrrolidinyl, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, piperazinyl, homopiperazinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinylimidazolinyl, imidazolidinyl, azetidin-2-one-1-yl, pyrrolidin-2-one-1-yl, piperid-2-one-1-yl, azepan-2-one-1-yl, 3-azabicyco[3.1.0]hexanyl, 3-azabicyclo [4.1.0]heptanyl, azabicyclo[2.2.2]hexanyl, 3H-indolyl and quinolizinyl. Spiro moieties are also included within the scope of this definition.

Carbocyclyl is a monocyclic or polycyclic ring system of 3 to 20 carbon atoms which may be saturated, unsaturated or aromatic.

Alkyl is a saturated hydrocarbon moiety, namely straight chain or branched alkyl having 1 to 10, preferably 1 to 8 carbon atoms, more preferably 1 to 4 carbon atoms, such as methyl, ethyl, n-propyl, isopropyl, n-butyl, isobutyl, tert-butyl, n-pentyl, isopentyl, neopentyl, hexyl or heptyl.

Cycloalkyl is an alkyl ring having 3 to 10, preferably 3 to 8 carbon atoms, more preferably 3 to 6 carbon atoms, such as cyclopropyl, cyclobutyl, cyclopentyl, cyclohexyl, cycloheptyl or cyclooctyl.

Alkenyl is an unsaturated hydrocarbon moiety with one or more double bonds, preferably one double bond, namely straight chain or branched alkenyl having 1 to 10, preferably 2 to 8 carbon atoms, more preferably 2 to 4 atoms, such as vinyl, allyl, methallyl, buten-2-yl, buten-3-yl, penten-2-yl, penten-3-yl, penten-4-yl, 3-methyl-but-3-enyl, 2-methyl-but-3-enyl, 1-methyl-but-3-enyl, hexenyl or heptenyl.

Alkynyl is an unsaturated hydrocarbon moiety with one or more triple bonds, preferably one triple bond, namely straight chain or branched alkynyl having 1 to 10, preferably 2 to 8 carbon atoms, more preferably 2 to 4 atoms, such as ethynyl, propynyl, butyn-2-yl, butyn-3-yl, pentyn-2-yl, pentyn-3-yl, pentyn-4-yl, 2-methyl-but-3-ynyl, 1-methyl-but-3-ynyl, hexynyl or heptynyl.

Halo or halogen is a halogen atom preferably selected from F, Cl, Br and I, preferably F, Cl and Br.

In the definitions cycloalkylalkyl, arylalkyl, heretoarylalkyl and heterocyclylalkyl it is contemplated that cycloalkyl, aryl, heretoaryl and heterocyclyl are bonded via an alkylene moiety. This alkylene moiety may be a straight chain or branched chain group. Said alkylene moiety preferably has 1 to 6 carbon atoms. Examples thereof include methylene, ethylene, n-propylene, n-butylene, n-pentylene, n-hexylene, iso-propylene, sec.-butylene, tert.-butylene, 1,1-dimethyl propylene, 1,2-dimethyl propylene, 2,2-dimethyl propylene, 1,1-dimethyl butylene, 1,2-dimethyl butylene, 1,3-dimethyl butylene, 2,2-dimethyl butylene, 2,3-dimethyl butylene, 3,3-dimethyl butylene, 1-ethyl butylene, 2-ethyl butylene, 3-ethyl butylene, 1-n-propyl propylene, 2-n-propyl propylene, 1-iso-propyl propylene, 2-iso-propyl propylene, 1-methyl pentylene, 2-methyl pentylene, 3-methyl pentylene and 4-methyl pentylene. More preferably, said alkylene moiety has 1 to 3 carbon atoms, such as methylene, ethylene, n-propylene and iso-propylene. Most preferred is methylene.

Preferred embodiments of the compounds according to present invention are shown in scheme 1.

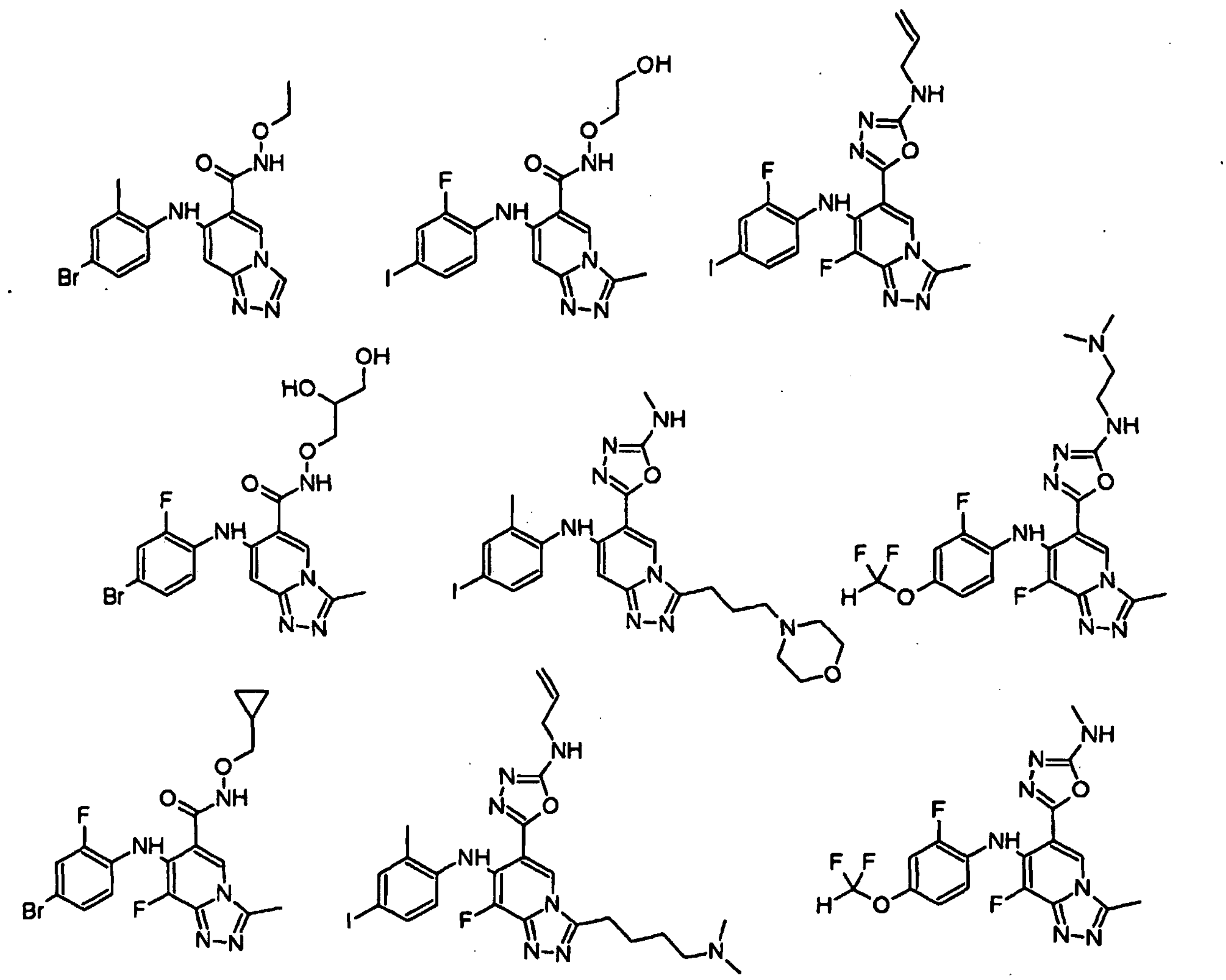

## Scheme 1

Where tautomerism, like e.g. keto-enol tautomerism, of compounds of the present invention or their prodrugs may occur, the individual forms, like e.g. the keto and enol form, are claimed separately and together as mixtures in any ratio. Same applies for stereoisomers, like e.g. enantiomers, cis/trans isomers, conformers and the like.

If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of the present invention may be obtained from stereoselective synthesis using optically pure starting materials.

The compounds of the present invention can be in the form of a pharmaceutically acceptable salt or a solvate. The term "pharmaceutically acceptable salts" refers to salts prepared from pharmaceutically acceptable non-toxic bases or acids, including inorganic bases or acids and organic bases or acids. In case the compounds of the present invention contain one or more acidic or basic groups, the invention also comprises their corresponding pharmaceutically or toxicologically acceptable salts, in particular their pharmaceutically utilizable salts. Thus, the compounds of the of the present invention which contain acidic groups can be present on these groups and can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the present invention which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic

acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the present invention simultaneously contain acidic and basic groups in the molecule, the invention also includes, in addition to the salt forms mentioned, inner salts or betaines (zwitterions). The respective salts can be obtained by customary methods which are known to the person skilled in the art like, for example by contacting these with an organic or inorganic acid or base in a solvent or dispersant, or by anion exchange or cation exchange with other salts. The present invention also includes all salts of the compounds of the present invention which, owing to low physiological compatibility, are not directly suitable for use in pharmaceuticals but which can be used, for example, as intermediates for chemical reactions or for the preparation of pharmaceutically acceptable salts.

Furthermore, the present invention provides pharmaceutical compositions comprising a compound of the present invention, or a prodrug compound thereof, or a pharmaceutically acceptable salt or solvate thereof as active ingredient together with a pharmaceutically acceptable carrier.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier.

A pharmaceutical composition of the present invention may additionally comprise one or more other compounds as active ingredients like one or more additional compounds of the present invention, or a prodrug compound or other MEK inhibitors.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In one embodiment, said compounds and pharmaceutical composition are for the treatment of cancer such as brain, lung, squamous cell, bladder, gastic, pancreatic, breast, head, neck, renal, kidney, ovarian, prostate, colorectal, oesohageal, testicular, gynecological or thyroid cancer. In another embodiment, said pharmaceutical composition is for the treatment of a noncancerous hyperproliferative disorder such as benign hyperplasia of the skin (e.g., psoriasis), restenosis, or prostate (e.g.benign prostatic hypertrophy (BPH)).

The invention also relates to a compound or pharmaceutical composition for the treatment of pancreatitis or kidney disease (including proliferative glomerulonephtitis and diabetes induced renal disease) or pain in a mammal which comprises a therapeutically effective amount of a compound of the present invention, or a pharmaceutically acceptable salt, prodrug or hydrate thereof, and a pharmaceutically acceptable carrier. The invention also relates to a compound or pharmaceutical composition for the prevention of blastocyte implantation in a mammal which comprises a therapeutically effective amount of a compound of the present invention, or a pharmaceutically acceptable salt, prodrug or hydrate thereof, and a pharmaceutically acceptable carrier. The invention also relates to a compound or pharmaceutical composition for treating a disease related to vasculogenesis or angiogenesis in a mammal which comprises a therapeutically effective amount of a compound of the present invention, or a pharmaceutically acceptable salt, prodrug or hydrate thereof, and a pharmaceutically acceptable carrier.

In one embodiment, said compound or pharmaceutical composition is for treating a disease selected from the group consisting of tumor angiogenesis, chronic inflammatory disease such as rheumatoid arthritis, inflammatory bowel disease, atherosclerosis, skin diseases such as psoriasis, eczema, and sclerodema, diabetes, diabetic retinopathy, retinopathy of prematurity, age-related macular degeneration, hemangioma, glioma, melanoma, Kaposi's sarcoma and ovarian, breast, lung, pancreatic, prostate, colon and epidermoid cancer.

The compounds of the invention can be used for treating a hyperproliferative disorder in a mammal. In one embodiment, said use relates to the treatment of cancer such as brain, lung, squamous cell, bladder, gastic, pancreatic, breast, head, neck, renal, kidney, ovarian, prostate, colorectal, oesohageal, testicular, gynecological or thyroid cancer. In another embodiment, said use relates to the treatment of a non-cancerous hyperproliferative disorder such as benign hyperplasia of the skin (e.g., psoriasis), restenosis, or prostate (e.g., benign prostatic hypertrophy (BPH)).

The compounds of the invention can be used for the treatment of a hyperproliferative disorder in a mammal, that comprises administering to said mammal a therapeutically effective amount of a compound of the present invention, or a pharmaceutically acceptable salt, or hydrate thereof, in combination with an anti-tumor agent selected from the group consisting of mitotic inhibitors, alkylating agents, antimetabolites, intercalating antibiotics, growth factor inhibitors, cell cycle inhibitors, enzyme inhibitors, topoisomerase inhibitors, biological response modifiers, antihormones, angiogenesis inhibitors, and anti-androgens.

The compounds of the invention can be used for treating pancreatitis or kidney disease or pain in a mammal that comprises administering to said mammal a therapeutically effective amount of a compound of the present invention, or a pharmaceutically acceptable salt or hydrate thereof. The invention also relates to a use of preventing blastocyte implantation in a mammal that comprises administering to said mammal a therapeutically effective amount of a compound of the present invention, or a pharmaceutically acceptable salt, prodrug or hydrate thereof.

The compounds of the invention can be used for treating diseases related to vasculogenesis or angiogenesis in a mammal that comprises administering to said mammal a therapeutically effective amount of a compound of the present invention, or a pharmaceutically acceptable salt or hydrate thereof. In one embodiment, said method is for treating a disease selected from the group consisting of tumor angiogenesis, chronic inflammatory disease such as rheumatoid arthritis, atherosclerosis, inflammatory bowel disease, skin diseases such as psoriasis, eczema, and scleroderma, diabetes, diabetic retinopathy, retinopathy of prematurity, age-related macular degeneration, hemangioma, glioma, melanoma, Kaposi's sarcoma and ovarian, breast, lung, pancreatic, prostate, colon and epidermoid cancer. Patients that can be treated with compounds of the present invention, or pharmaceutically acceptable salts, prodrugs and hydrates of said compounds, according to the methods of this invention include, for example, patients that have been diagnosed as having psoriasis, restenosis, atherosclerosis, BPH, lung cancer, bone cancer, CMML, pancreatic cancer, skin cancer, cancer of the head and neck, cutaneous or intraocular melanoma, uterine cancer, ovarian cancer, rectal cancer, cancer of the anal region, stomach cancer, colon cancer, breast cancer, testicular, gynecologic tumors (e.g., uterine sarcomas, carcinoma of the fallopian tubes, carcinoma of the endometrium, carcinoma of the cervix, carcinoma of the vagina or carcinoma of the vulva), Hodgkin's disease, cancer of the esophagus, cancer of the small intestine, cancer of the endocrine system (e.g., cancer of the thyroid, parathyroid or adrenal glands), sarcomas of soft tissues, cancer of the urethra, cancer of the penis, prostate cancer, chronic or acute leukemia, solid tumors of childhood, lymphocytic lymphomas, cancer of the bladder, cancer of the kidney or ureter (e.g., renal cell carcinoma, carcinoma of the renal pelvis), or neoplasms of the central nervous system (e.g., primary CNS lymphona, spinal axis tumors, brain stem gliomas or pituitary adenomas).

This invention also relates to a compound or pharmaceutical composition for inhibiting abnormal cell growth in a mammal which comprises an amount of a compound of the present invention, or a pharmaceutically acceptable salt or solvate or prodrug thereof, in combination with an amount of a chemotherapeutic, wherein the amounts of the compound, salt, solvate, or prodrug, and of the chemotherapeutic are together effective in inhibiting abnormal cell growth. Many chemotherapeutics are presently known in the art. In one embodiment, the chemotherapeutic is selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, anti-hormones, angiogenesis inhibitors, and anti-androgens. This invention further relates to a method for inhibiting abnormal cell growth in a mammal or treating a hyperproliferative disorder which method comprises administering to the mammal an amount of a compound of the present invention, or a pharmaceutically acceptable salt or solvate or prodrug thereof, in combination with radiation therapy, wherein the amounts of the compound, salt, solvate, or prodrug, is in combination with the radiation therapy effective in inhibiting abnormal cell growth or treating the hyperproliferative disorder in the mammal. Techniques for administering radiation therapy are known in the art, and these techniques can be used in the combination therapy described herein. The administration of the compound of the invention in this combination therapy can be determined as described herein. It is believed that the compounds of the present invention can render abnormal cells more sensitive to treatment with radiation for purposes of killing and/or inhibiting the growth of such cells. Accordingly, this invention further relates to a method for sensitizing abnormal cells in a mammal to treatment with radiation which comprises administering to the mammal an amount of a compound of the present invention or pharmaceutically acceptable salt or solvate or prodrug thereof, which amount is effective is sensitizing abnormal cells to treatment with radiation. The amount of the compound, salt, or solvate in this method can be determined according to the means for ascertaining effective amounts of such compounds described herein. The invention also relates to a method of and to a pharmaceutical composition of inhibiting abnormal cell growth in a mammal which comprises an amount of a compound of the present invention, or a pharmaceutically acceptable salt or solvate thereof, a prodrug thereof, or an isotopically-labeled derivative thereof, and an amount of one or more substances selected from anti-angiogenesis agents, signal transduction inhibitors, and antiproliferative agents.

In practical use, the compounds of the present invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets,

with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin.

When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of the present invention may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of the present invention are administered orally.

The effective dosage of active ingredient employed may vary depending on the particular compound employed, the mode of administration, the condition being treated and the severity of the condition being treated. Such dosage may be ascertained readily by a person skilled in the art.

When treating or preventing cancer, inflammation or other proliferative diseases for which compounds of the present invention are indicated, generally satisfactory results are obtained when the compounds of the present invention are administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams, preferably from about 1 milligram to about 50 milligrams. In the case of a 70 kg adult human, the total daily dose will generally be from about 7 milligrams to about 350 milligrams. This dosage regimen may be adjusted to provide the optimal therapeutic response.

Some abbreviations that may appear in this application are as follows.

***Abbreviations***

*Designation*

| | |
|---|---|
| b | Broad peak |
| Boc | *tert.*-Butyloxycarbonyl |
| CDI | N,N-Carbonyldiimidazole |
| d | Doublet |
| DCM | Dichloromethane |

| | |
|---|---|
| dd | double doublet |
| DIPEA | N-Ethyldiisopropylamine |
| DMF | N,N-Dimethylformamide |
| DMSO | Dimethylsulfoxide |
| EDC | 1-(3-Dimethylaminopropyl)-3-ethylcarbodiimide hydrochloride |
| HPLC | High pressure liquid chromatography |
| LiHMDS. | Lithium hexamethyldisilazide |
| NMR | Nuclear Magnetic Resonance |
| PG | Protecting group |
| PyBroP | Bromo-tris-pyrrolidino-phosphonium hexafluorophosphate |
| PyBOP | Benzotriazole-1-yl-oxy-trispyrrolidinophosphonium hexafluorophosphate |
| PPA | Polyphosphoric acid |
| q | Quartett |
| rt | Retention time |
| s | Singlet |
| tert | Tertiary-butyl |
| TFA | Trifluoroacetic acid |
| THF | Tetrahydrofurane |
| TLC | Thin Layer Chromatography |

The compounds of the present invention can be prepared according to the procedures of the following Schemes and Examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described above. The amine-free bases corresponding to the isolated salts can be generated by neutralization with a suitable base, such as aqueous sodium hydrogencarbonate, sodium carbonate, sodium hydroxide and potassium hydroxide, and extraction of the liberated amine-free base into an organic solvent, followed by evaporation. The amine-free base, isolated in this manner, can be further converted into another pharmaceutically acceptable salt by dissolution in an organic solvent, followed by addition of the appropriate acid and subsequent evaporation, precipitation or crystallisation.

An illustration of the preparation of compounds of the present invention is shown in schemes 2 and 3. Unless otherwise indicated in the schemes, the variables have the same meaning as described above.

The examples presented below are intended to illustrate particular embodiments of the invention.

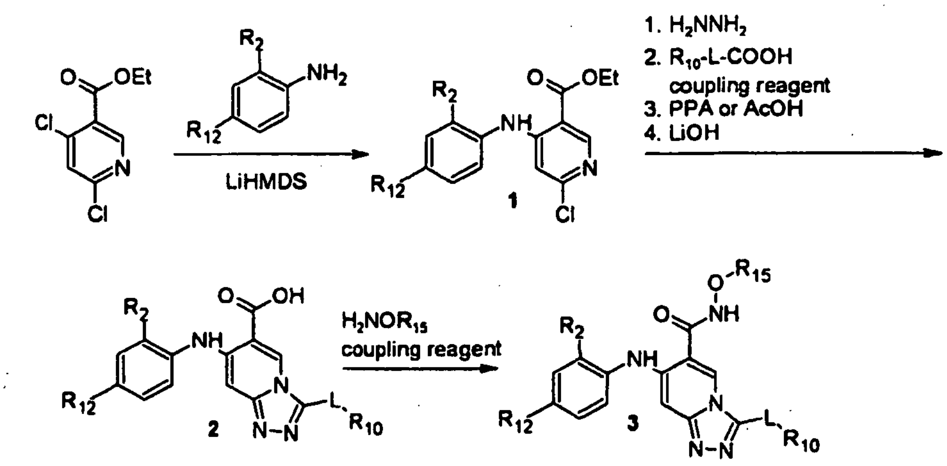


Scheme 2

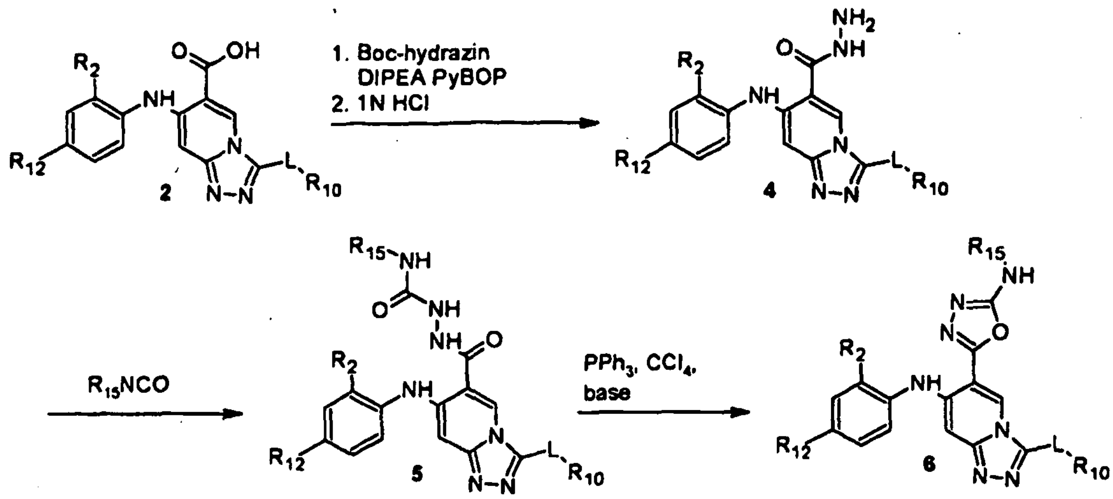


Scheme 3

As outlined in scheme2, ethyl 4,6-dichloronicotinate can be reacted with an appropriately substituted aniline in an inert solvent, preferable THF, by addition of a base, preferably but not limited to LiHMDS to yield substituted ethyl 6-chloro-4arylaminonicotinate **1.** Heating with hydrazine hydrate leads to an intermediate hydrazino compound which is acylated by coupling with an activated carboxylic acid building block. Cyclisation is achieved by heating in acidic solvents such as AcOH or PPA. The resulting triazolopyridine is saponified in the next step by heating with lithium hydroxide in a water-alcohol mixture to give triazolopyridine carboxylic acid **2.** The latter can be further derivatised by coupling with a substituted O-alkylhydroxylamine using a coupling reagent such as PyBro, PyBOP or DCC for example to give substituted hydroxamate **3.**

Scheme 3 illustrates the preparation of compounds of the present invention where W is heterocyclic. Triazolopyridine carboxylic acid **2** can be coupled with Boc-hydrazine by using a coupling reagent such as PyBOP or DCC for example and the Boc protecting group is then removed by treatment with an acid such as HCl. The resulting hydrazide **4** is further derivatised by reacting with an appropriately substituted isocyanate. Cyclisation can be achieved by heating with triphenyl phosphine and $CCl_4$ in a suitable solvent to give oxadiazole compound **6.**

Suitable anilines, carboxylic acids, O-alkyl hydroxylamines, and isocyanates are commercially available from Sigma-Aldrich Chemie GmbH, Munich, Germany or from Acros Organics, Belgium or from Fisher Scientific GmbH, 58239 Schwerte, Germany or can be routinely prepared by procedures described in "March's Advanced Organic Chemistry: Reactions, Mechanisms, and Structure", 5th Edition; John Wiley & Sons. O-[(4*S*)2,2-Dimethyl-[1,3]dioxolan-4-ylmethyl]-hydroxylamine and O-[(4*R*)2,2-dimethyl-[1,3]dioxolan-4-ylmethyl]-hydroxylamine are prepared according to a

procedure described in WO02/06213 A2. Ethyl 4,6-dichloronicotinate is synthesised in three steps from diethyl acetone dicarboxylate according to a literature procedure (DenHertog, Recl Trav Chim Pays-Bas 1946, 65, 129-140).

Compounds with other variants in the position of W can be prepared by derivatizing the COOH group appropriately as known to the person skilled in the art as described in Theophil Eicher, Siegfried Hauptmann "The Chemistry of Heterocycles; Structures, Reactions, Synthesis and Application", 2nd edition, Wiley-VCH 2003. The introduction of alternative heterocyclic or heteroaryl groups is exemplified e.g. in WO 03/077855 and WO 01/05391.

Unless otherwise noted, all non-aqueous reactions were carried out either under an argon or nitrogen atmosphere with commercial dry solvents. Compounds were purified using flash column chromatography using Merck silica gel 60 (230-400 mesh), or by reverse phase preparative HPLC using a Reprosil-Pur ODS3, 5 μm, 20 x 125 mm column with Shimadzu LC8A-Pump and SPD-10Avp UV/Vis diode array detector. The $^{1}$H-NMR spectra were recorded on a Varian VXR-S (300 MHz for $^{1}$H-NMR) using $d_6$-dimethylsulfoxide or $d_4$-methanol as solvent; chemical shifts are reported in ppm relative to tetramethylsilane. Analytical LC/MS was performed using Reprosil-Pur ODS3, 5 μM, 1 x 60 mm columns at a flow rate of 250 μl/min, sample loop 2.5μl; retention times are given in minutes. Methods are: (I) runs on a LC10Advp-Pump (Shimadzu) with SPD-M10Avp UV/Vis diode array detector and QP2010 MS-detector in ESI+ modus with UV-detection at 214, 254 and 275 nm with a gradient of 15-95% acetonitrile (B) in water (A) (0.1 % formic acid), 5 min. linear gradient; (II) idem but linear gradient 8min 1-30% B; (III) idem but linear gradient 8min 10-60% B; (IV) idem but linear gradient 8min 15-99% B; (V) idem but linear gradient 10min 5-95% B; (VI) idem but linear gradient 10min 10-95% B; (VII) idem but linear gradient 5min 10-90% B; (VIII) idem but linear gradient 5min 5-95% B.

***Example 1***

***7-[(4-Bromo-2-methylphenyl)amino]-3-methyl[1,2,4]triazolo[4,3-a]pyridine-6-carboxylic acid* (2a)**

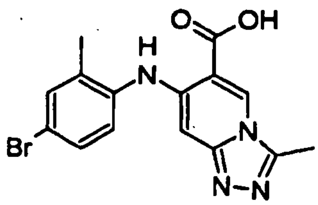

**Step A**

Ethyl 4,6-dichloronicotinate (2.0g, 9.1mmol) and 4-bromo-2-methylaniline (1.7g, 9.1mmol) are dissolved in dry THF (20ml) under argon and the mixture is cooled to -78°C. A solution of LiHMDS (1.0M in THF, 32ml) is slowly added and the reaction mixture is allowed to warm to ambient temperature. After 18h the reaction is quenched by adding dilute hydrochloric acid (10M, 20.0ml) and the mixture is extracted with DCM (3x 60ml). The combined organic extracts are concentrated *in vacuo* and the crude material is purified by flash chromatography using silica gel and a gradient of 0-10% ethylacetate in cyclohexane as eluent to give pure ethyl 4-[(4-bromo-2-methylphenyl)amino]-6-chloronicotinate **(1a)** (900mg, 27% yield).
LC-MS method (I) rt 5.34 min; m/z 369, 371 $[M+H]^+$, Br, Cl pattern.
$^{1}$H-NMR (300MHz, $CDCl_3$): δ = 1.42 (t, J = 7.3Hz, 3H), 2.21 (s, 3H), 4.39 (q, J = 7.3Hz, 2H), 6.50 (s, 1H), 7.11 (d, J = 8.1Hz, 1H), 7.35-7.41 (m, 1H), 7.44-7.47 (m, 1H), 8.76 (s, 1H), 9.59 (b, 1 H)

**Step B**

Compound **1a** (500mg, 1.35mmol) is dissolved in dry dioxane (10m), hydrazine hydrate (985μl, 20.3mmol) is added and the mixture is heated at 150°C with microwave irradiation for 30min. The volatiles are removed *in vacuo* and the crude ethyl 4-[(4-bromo-2-methylphenyl)amino]-6-hydrazinonicotinate is used in the next step.
LC-MS method (I) rt 2.26 min; m/z 365, 367 $[M+H]^+$, Br pattern.

**Step C**

4-[(4-Bromo-2-methylphenyl)amino]-6-hydrazinonicotinate (crude from step B, ~1.35mmol) is dissolved in dry pyridine (8ml) and acetic anhydride (4ml) and the mixture is heated at 60°C for 20 min. The volatiles are removed *in*

*vacuo,* acetic acid (5ml) and acetic anhydride (2.5ml) are added and the mixture is heated for 90min at 170°C with microwave irradiation. The volatiles are removed *in vacuo,* the residue is redissolved in THF (8ml), water (0.8ml) and LiOH (2.1mmol, 71mg) is added and the mixture is heated to 120°C with microwave irradiation for 20minutes. The volatiles are removed *in vacuo,* the residual material is adsorbed on flash silica and purified by flash chromatography using silica gel and a gradient of 0-10% methanol in DCM as eluent to give 361 mg (1.0 mmol, 74%yield for steps B and C) of pure 7-[(4-Bromo-2-methylphenyl)amino]-3-methyl[1,2,4]triazolo[4,3-a]pyridine-6-carboxylic acid **2a.**
LC-MS method (V) rt 1.86 min; m/z 361, 363 $[M+H]^+$, Br pattern.
$^1$H-NMR (300MHz, DMSO-$d_6$): δ = 2.22 (s, 3H), 2.60 (s, 3H), 6.59 (s, 1H), 7.40-7.50 (m, 3H), 8.60 (s, 1 H).

***Example 2***

***7-[(4-Bromo-2-methylphenyl)amino]-N-hydroxy-3-methyl[1,2,4]triazolo[4,3-a]pyridine-6-carboxamide*** **(3a)**

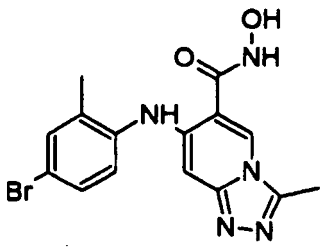

7-[(4-Bromo-2-methylphenyl)amino]-3-methyl[1,2,4]triazolo[4,3-a]pyridine-6-carboxylic acid (2a) (50mg, 0.138mmol) is dissolved in dry DMF and hydroxylamine hydrochloride (19.2mg, 0.277mmol), PyBrOP (83.9mg, 0.180mmol) and DIPEA (96μl, 0.554mmol) is added. The mixture is stirred for 6h, the volatiles are evaporated and the crude material is purified by preparative HPLC to give 30 mg (0.080mmol, 58%yield) of pure **3a**.
LC-MS method (VII) rt 1.92 min; m/z 376, 378 $[M+H]^+$, Br pattern.
$^1$H-NMR (400MHz, MeOH-$d_4$): δ = 2.28 (s, 3H), 2.75 (s, 3H), .6.42 (s, 1 H), 7.30 (d, J = 8.6Hz, 1 H), 7.50-7.55 (m, 1H), 7.63 (s, 1H), 8.79 (s, 1 H).

***Example 3***

***7-[(4-Bromo-2-methylphenyl)amino]-N-{[(2S)-2,3-dihydroxypropyl]oxy}-3-methyl[1,2,4]triazolo[4,3-a]pyridine-6-carboxamide*** **(3b)**

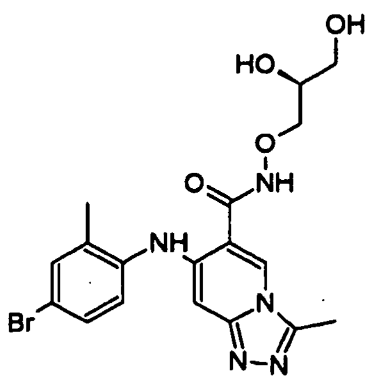

**Step A**

7-[(4-Bromo-2-methylphenyl)amino]-3-methyl[1,2,4]triazolo[4,3-a]pyridine-6-carboxylic acid **(2a)** (50mg,

0.138mmol) is dissolved in dry DMF (2.0ml) and DIPEA (96µl, 0.554mmol), PyBroP (84mg, 0.180mmol) and *O*-{[(4*S*)-2,2-dimethyl-1,3-dioxolan-4-yl]methyl}hydroxylamine (27mg, 0.18mmol) are added. The mixture is stirred for 6h at ambient temperature and the volatiles are evaporated. DCM is added and the mixture is washed with saturated $NaHCO_3$ solution, dried ($Na_2SO_4$) and evaporated to give the crude hydroxamate which is used without purification in the next step. LC-MS method (VII) rt 2.64 min; m/z 490, 492 $[M+H]^+$, Br pattern.

**Step B**

The crude material from step A is dissolved in MeOH (1.8m) and water (0.2ml). Dowex50X8 resin (20mg) is added and the mixture is heated to 120°C with microwave irradiation for 20min, the solution is filtered and evaporated. The crude material is purified by preparative HPLC to give 18.6mg (41.4µmol, 30%yield, two steps) of pure hydroxamate **3b.**

LC-MS method (II) rt 6.01 min; m/z 450, 452 $[M+H]^+$, Br pattern.

$^1$H-NMR (400MHz, MeOH-$d_4$): δ = 2.27 (s, 3H), 2.70 (s, 3H), 3.62-3.66 (m, 2H), 3.92-3.99 (m, 1H), 4.01-4.07 (m, 1H), 4.14-4.19 (m, 1H), 6.43 (s, 1H), 7.27 (d, J = 8.6Hz, 1 H), 7.40-7.46 (m, 1H), 7.52 (s, 1H), 8.56 (s, 1H).

***Example 4***

***7-[(4-iodo-2-methylphenyl)amino]-N-{[(2R)-2,3-dihydroxypropyl]oxy}-3-methyl[1,2,4]triazolo[4,3-a]pyridine-6-carboxamide (3c)***

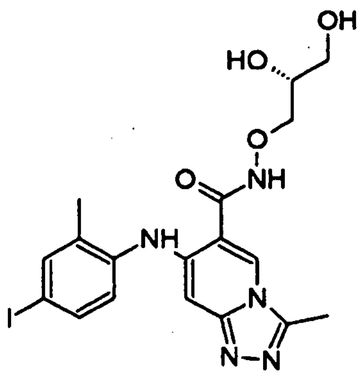

**Step A**

Ethyl 4,6-dichloronicotinate (1) (3.0g, 14mmol) and 4-iodo-2-methylaniline (3.2g, 14mmol) are dissolved in dry THF (20ml) under argon and the mixture is cooled to -78°C. A solution of LiHMDS (1.0M in THF, 48ml) is slowly added and the reaction mixture is allowed to warm to ambient temperature. The reaction is quenched after 20h by adding dilute hydrochloric acid (1.0M, 20.0ml), the volatiles are evaporated and the residue is extracted with DCM (3x 60ml). The combined organic extracts are concentrated *in vacuo* and the crude material is purified by flash chromatography using silica gel and a gradient of 0-10% ethylacetate in cyclohexane as eluent to give pure ethyl 4-[(4-iodo-2-methylphenyl) amino]-6-chloronicotinate (700mg, 12% yield).

LC-MS method (VIII) rt 5.64 min; m/z 417 $[M+H]^+$, Cl pattern.

$^1$H-NMR (400MHz, DMSO-$d_6$): δ = 1.33 (t, J = 7.1 Hz, 3H), 2.13 (s, 3H), 4.35 (q, J = 7.1Hz, 2H), 6.41 (s, 1H), 7.12 (d, J = 8.6Hz, 1H), 7.63 (d, J = 8.1Hz, 1H), 7.76 (s, 1H), 8.65 (s, 1H), 9.52 (b, 1 H).

**Step B**

4-[(4-lodo-2-methylphenyl)amino]-6-chloronicotinate (350mg, 0.84mmol) is dissolved in dry dioxane (7ml), hydrazine hydrate (611µl, 12.6mmol) is added and the mixture is heated at 150°C with microwave irradiation for 30min. The volatiles are removed *in vacuo* and the crude ethyl 4-[(4-iodo-2-methylphenyl)amino]-6-hydrazinonicotinate is directly used in the next step.

LC-MS method (VII) rt 2.68 min; m/z 413 $[M+H]^+$.

**Step C**

4-[(4-iodo-2-methylphenyl)amino]-6-hydrazinonicotinate (crude from step B, ~0.84mmol) is dissolved in dry pyridine (8ml) and acetic anhydride (4ml) and the mixture is heated at 70°C for 20 min with microwave irradiation. The volatiles are removed *in vacuo,* acetic acid (3ml) and acetic anhydride (1.5ml) are added and the mixture is heated for 90min at 170°C with microwave irradiation. The volatiles are removed *in vacuo* and the crude material is purified by flash-chomatography using silica gel and 5% methanol in DCM as eluent to give 130mg (0.30mmol, 35%yield for steps B and C) ethyl 7-[(4-iodo-2-methylphenyl)amino]-3-methyl[1,2,4]triazolo[4,3-a]pyridine-6-carboxylate.
LC-MS method (VII) rt 2.96 min; m/z 437 $[M+H]^+$.

**Step D**

Ethyl 7-[(4-iodo-2-methylphenyl)amino]-3-methyl[1,2,4]triazolo[4,3-a]pyridine-6-carboxylate (130mg, 0.298mmol) is dissolved in THF (6ml), water (1ml) and LiOH hydrate (68mg, 1.62mmol) are added and the mixture is heated for 30 min at 120°C with microwave irradiation. Hydrochloric acid (1 N in dioxane, 4ml) is added and the volatiles are removed under reduced pressure. The residue is partitioned between DCM and water, the organic phase is dried ($Na_2SO_4$) and evaporated to give crude 7-[(4-iodo-2-methylphenyl)amino]-3-methyl[1,2,4]triazolo[4,3-a]pyridine-6-carboxylic acid.
LC-MS method (VII) rt 2.60 min; m/z 409 $[M+H]^+$.

**Step E**

The crude carboxylic acid from step D (~0.27mmol) is dissolved in dry DMF, ByBrOP (162mg, 0.348mmol), DIPEA (138mg, 1.07mmol) and *O*-{[(4*R*)-2,2-dimethyl-1,3-dioxolan-4-yl]methyl}hydroxylamine (51mg, 0.35mmol) are added and the mixture is stirred at 50°C over night. The volatiles are evaporated, DCM is added and washed with pH7-phosphate buffer and brine, dried ($Na_2SO_4$) and evaporated. The residue is dissolved in methanol (2.7ml) and water (0.3ml) and Dowex50X8 resin (18mg) is added. The mixture is heated at 120°C for 40min with microwave irradiation, filtered and evaporated. The crude product is purified by preparative HPLC to yield 30mg of hydroxamate **3c**.

***Assay***

The activity of the compounds of the present invitation may be determined by the following procedure: Inhibition of human MEK1 kinase activity was monitored with a homogenous, fluorescence based assay. The assay uses time resolved fluorescence resonance energy transfer to probe for phosphorylation of ERK1 by MEK1. The assay is carried out in low volume 96 well microtiterplates. In a total volume of 15 μl, compounds are incubated with 100nM MEK1, 15 μM ATP, 300nM ERK2 employing a buffer containing 20mM TRIS/HCl, 10 mM MgCl2, 100 μM NaV04, 1 mM DTT, and 0.005% Tween 20 (pH 7.4). After two hours, 5 nM Europium-anti-PY20 (Perkin Elmer) and 50nM Anti-GST-Allophycocyanin (CisBio) in buffer containing 50mM EDTA and 0,05% BSA are added and the reaction incubated for one hour in the dark. Time-resolved fluorescence is measured using a LJL-Analyst (Molecular Devices) with an excitation wavelength of 340 nm and an emission wavelength of 665 nm. The final concentration of DMSO is 2 %. To assess the inhibitory potential of the compounds, IC50-values were determined.

In this assay compounds of the invention exhibited IC50s within certain ranges. The following compounds exemplify such activity with "+" meaning 1μM < IC50 ≤ 10μM and "++" IC50 ≤ 1μM

**Table 1:** Inhibition of MEK

| Compound # | Activity |
|---|---|
| 2a | + |
| 3a | ++ |
| 3b | + |
| 3c | ++ |

***Assay 2: Tumor cell proliferation assays (A TP Lite)***

Murine colon C26, human melanoma A375 and human melanoma Mel5 cells were plated in 96 well Coming white plates (1500 cells/well for C26, and 2000 cells/well for A375, and MiaPaCa-2) and cultured overnight at 37°C in

5% CO2. Inhibitors were serially diluted in 100 % DMSO and subsequently added to cells to reach a final concentration of 0.25% DMSO. The cells were incubated for 4 days in the presence of test compounds in cell growth media (DMEM with 10% fetal bovine serum, 2mM glutamine for C26, and MiaPaCa-2, and RPMI with 10% fetal bovine serum, 2mM glutamine for A375). Cell proliferation was quantitated using the ATP lite cell proliferation kit (Packard). Inhibition of cell proliferation is shown in Table 2. Columns 2-4 show the concentration of compounds required to induce 50% cell death (IC50 in μM) of human endometriotic cells. With "+" meaning 100μM < IC50 ≤ 10μM and "++" IC50 ≤ 1 μM and "n.d." means not determined.

***Assay 3*: *Microsomal stability assay***

Compounds were tested on their stability in human, rat and mouse liver microsomal preparations (HLM, RLM and MLM respectively). At a final concentration of 3 μM, compounds were incubated at 37°C with 0.5 mg/ml human, rat or mouse liver microsomes in a buffer containing 50 mM phosphate, pH 7.4 and 2 mM NADPH. Pooled human liver microsomes or pooled male rat liver microsomes (Sprague Dawley) were obtained from NatuTec (Frankfurt, Germany). Incubations without NADPH served as negative controls. Reactions were stopped after 0, 15, 30, 45 or 60 min by the addition of acetonitrile and microsomes were pelleted by centrifugation (10 min at 6200 x g). Supernatants were analyzed by HPLC regarding the concentration of mother compound. Finally, the half-life of compounds in the regarding microsomal preparation was calculated. Results are shown in Table 2. Wherein "+" means $t_{1/2}$ of 1-30 min, "++" means $t_{1/2}$ of 31-120 min and "+++" means $t_{1/2}$ of > 120 min and "n.d." means not determined.

***Assay 4: Caco-2 permeability assay***

Caco-2 cells obtained from the ATCC at passage number 27 are used. Cells (passage number 40-60) were seeded on to Millipore Multiscreen Caco-2 plates or Falcon HTS inserts at 1 x 105 cells/cm2. Cells were cultured for 20 days in DMEM and media was changed every two or three days. On day 20 the permeability study was performed.

Permeability was studied by applying compound to the apical surface of cell monolayers and measuring compound permeation into the basolateral compartment. The experiment was also performed in the reverse direction (B-A) to investigate active transport. Hanks Balanced Salt Solution (HBSS) pH 7.4 buffer with 25mM HEPES and 10mM glucose at 37°C was used as the medium in permeability studies. Incubations were carried out in an atmosphere of 5% $CO_2$ with a relative humidity of 95%.

The monolayers were prepared by rinsing both basolateral and apical surfaces twice with HBSS at 37°C. Cells were then incubated with HBSS in both apical and basolateral compartments for 40 minutes to stabilize physiological parameters.

HBSS was then removed from the apical compartment and replaced with test compound dosing solutions. The solutions were made by diluting 10mM DMSO concentrates with HBSS to give a final test compound concentration of 10μM (final DMSO concentration adjusted to 1 %). The fluorescent integrity marker lucifer yellow was also included in the dosing solution: Analytical standards were made from dosing solutions. Test compound permeability was assessed in duplicate. On each plate compounds of known permeability characteristics were run as controls.

The apical compartment inserts were then placed into 'companion' plates containing fresh HBSS. For basolateral to apical (B-A) experiments the experiment was initiated by replacing buffer in the inserts then placing them in companion plates containing dosing solutions. At 120 minutes the companion plate was removed and apical and basolateral samples diluted for analysis by LC-MS/MS (the donor compartment was also sampled to permit determination of starting concentration after non-specific binding has occurred).

Analysis

The integrity of the monolayers throughout the experiment is checked by monitoring lucifer yellow permeation using fluorimetric analysis. Lucifer yellow permeation was low if monolayers have not been damaged. Test and control compounds were quantified by LC-MS/MS cassette analysis using a 5-point calibration with appropriate dilution of the samples. Should lucifer yellow Papps were above QC limits in more than one well per test compound, the compound was re-tested.

The permeability coefficient for each compound ($P_{app}$) was calculated from the following equation:

$$P_{app} = [dQ/dt]/[C_0 \times A]$$

Whereby dQ/dt is the rate of permeation of the drug across the cells, $C_0$ is the donor compartment concentration

at time zero and A is the area of the cell monolayer. $C_0$ is obtained from analysis of the donor compartment at the end of the incubation period.

Test compounds were grouped into low, medium or high absorption potential based on comparison with control compounds, which have known human absorption.

In addition, permeation was studied in both directions across the cells, and an asymmetry index was reported from mean A-B and B-A data. This was derived from:

$$P_{app\ (B-A)}/P_{app\ (A-B)}$$

Results are shown in Table 2. Wherein "+" means a caco A-B and caco B-A value of 1-10 and "++"means a caco A-B and caco B-A value of 11-100 and "n.d." means not determined.

**Table 2:** Results of inhibition of tumor cell proliferation and microsomal stability

| Compound No. | C26 cells IC50[uM] | Mel5 cells IC50 [uM] | A375 cells IC50 [uM] | HLM t1/2 [min] | RLM t1/2 [min] | Caco A-B [$10^{-6}$ cm/s] | Caco B-A [$10^{-6}$ cm/s] |
|---|---|---|---|---|---|---|---|
| 2a | + | + | + | n.d. | n.d. | n.d. | n.d. |
| 3a | + | + | + | n.d. | n.d. | n.d. | n.d. |
| 3b | + | + | + | +++ | +++ | + | + |

## Claims

**1.** A compound of formula (I),

Formula (I)

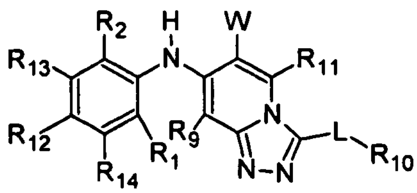

a pharmaceutically acceptable salt or solvate thereof, wherein:

$R_1$, $R_2$, $R_9$, $R_{11}$ $R_{12}$, $R_{13}$ and $R_{14}$ are independently selected from hydrogen, halogen, cyano, nitro, azido, $-OR_3$, $-C(O)R_3$,$-C(O)OR_3$, $-NR_4C(O)OR_6$, $-OC(O)R_3$, $-NR_4S(O)_jR_6$, $-S(O)_jNR_3R_4$, $-S(O)_jNR_4C(O)R_3$, $-C(O)NR_4S(O)_jR_6$, $S(O)_jR_6$, $-NR_4C(O)R_3$, $-C(O)NR_3R_4$, $-NR_5C(O)NR_3R_4$, $-NR_5C(NCN)NR_3R_4$, $-NR_3R_4$ and $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, $-S(O)_jC_1$-$C_6$ alkyl), $-S(O)_j(CR_4R_5)_m$-aryl, aryl, arylalkyl, heteroaryl, heteroarylalkyl, heterocyclyl, heterocyclylalkyl, $-O(CR_4R_5)_m$-aryl, $-NR_4(CR_4R_5)_m$-aryl, $-O(CR_4R_5)_m$-heteroaryl, $-NR_4(CR_4R_5)_m$-heteroaryl, $-O(CR_4R_5)_m$-heterocyclyl, $-NR_4(CR_4R_5)_m$-heterocyclyl, and $-S(C_1$-$C_2$ alkyl) substituted with 1 to 5 F, where each alkyl, alkenyl, alkynyl, cycloalkyl, aryl, heteroaryl and heterocyclyl is unsubstituted or substituted with 1 to 5 groups independently selected from oxo, halogen, cyano, nitro, $CF_3$, $CHF_2$, $CH_2F$, $OCF_3$, $OCHF_2$, $OCH_2F$, $SCF_3$, $SCHF_2$, $SCH_2F$, azido, $NR_4SO_2R_6$, $SO_2NR_3R_4$, $C(O)R_3$, $C(O)OR_3$, $OC(O)R_3$, $NR_4C(O)OR_6$, $NR_4C(O)R_3$, $C(O)NR_3R_4$, $NR_3R_4$, $NR_5C(O)NR_3R_4$, $NR_5C(NCN)NR_3R_4$, $OR_3$, aryl, heteroaryl, arylalkyl, heteroarylalkyl, heterocyclyl, and heterocyclylalkyl;

$R_{10}$ is selected from hydrogen, $-OR_3$, $-C(O)R_3$,$-C(O)OR_3$, $-NR_4C(O)OR_6$, $-OC(O)R_3$, $-NR_4S(O)_jR_6$ , $-S(O)_jNR_3R_4$, $-S(O)_jNR_4C(O)R_3$, $-C(O)NR_4S(O)_jR_6$, $S(O)_jR_6$, $-NR_4C(O)R_3$, $-C(O)NR_3R_4$, $-NR_5C(O)NR_3R_4$, $-NR_5C(NCN)NR_3R_4$, $-NR_3R_4$ ; $-S(O)_j(C_1$-$C_6$ alkyl), $-S(O)_j(CR_4R_5)_m$-aryl, $-O(CR_4R_5)_m$-aryl, $-NR_4(CR_4R_5)_m$-aryl, $-O(CR_4R_5)_m$-heteroaryl, $-NR_4(CR_4R_5)_m$-heteroaryl, $-O(CR_4R_5)_m$-heterocyclyl,- $NR_4(CR_4R_5)_m$-heterocyclyl, and $-S(C_1$-$C_2$ alkyl) substituted with 1 to 5 F, where each aryl, heteroaryl and heterocyclyl is unsubstituted or substituted with 1 to 5 groups independently selected from oxo, halogen, cyano, nitro, $CF_3$, $CHF_2$, $CH_2F$, $OCF_3$, $OCHF_2$, $OCH_2F$, $SCF_3$, $SCHF_2$, $SCH_2F$, azido, $NR_4SO_2R_6$, $SO_2NR_3R_4$, $C(O)R_3$, $C(O)OR_3$, $OC(O)R_3$, $NR_4C(O)OR_6$, $NR_4C(O)R_3$, $C(O)NR_3R_4$, $NR_3R_4$, $NR_5C(O)NR_3R_4$, $NR_5C(NCN)NR_3R_4$, $OR_3$, aryl, heteroaryl, arylalkyl,

heteroarylalkyl, heterocyclyl, and heterocyclylalkyl;

L is selected from a bond, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, aryl, arylalkyl, heteroaryl, heteroarylalkyl, heterocyclyl, heterocyclylalkyl, where each alkyl, alkenyl, alkynyl, cycloalkyl, aryl, heteroaryl and heterocyclyl is unsubstituted or substituted with 1 to 5 groups independently selected from oxo, halogen, cyano, nitro, $CF_3$, $CHF_2$, $CH_2F$, $OCF_3$, $OCHF_2$, $OCH_2F$, $SCF_3$, $SCHF_2$, $SCH_2F$, azido, $NR_4SO_2R_6$, $SO_2NR_3R_4$, $C(O)R_3$, $C(O)OR_3$, $OC(O)R_3$, $NR_4C(O)OR_6$, $NR_4C(O)R_3$, $C(O)NR_3R_4$, $NR_3R_4$, $NR_5C(O)NR_3R_4$, $NR_5C(NCN)NR_3R_4$, $OR_3$, aryl, heteroaryl, arylalkyl, heteroarylalkyl, heterocyclyl, and heterocyclylalkyl;

$R_3$ is selected from hydrogen, trifluoromethyl, $C_1$-$C_{10}$ alkyl, $C_{2-10}$ alkenyl, $C_2$-$C_{10}$ alkynyl, $C_3$-$C_{10}$ cycloalkyl, $C_3$-$C_{10}$ cycloalkylalkyl, aryl, arylalkyl, heteroaryl, heteroarylalkyl, heterocyclyl, and heterocyclylalkyl, where each alkyl, alkenyl, alkynyl, cycloalkyl, aryl, heteroaryl and heterocyclyl is unsubstituted or substituted with 1 to 5 groups independently selected from oxo, halogen, cyano, nitro, $CF_3$, $CHF_2$, $CH_2F$, $OCF_3$, $OCHF_2$, $OCH_2F$, azido, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''', OR', aryl, heteroaryl, arylalkyl, hetero-arylalkyl, heterocyclyl, and heterocyclylalkyl;

$R_4$ is selected from hydrogen or $C_1$-$C_6$ alkyl whereby alkyl may be unsubstituted or substituted with 1 to 5 groups independently selected from oxo, halogen, cyano, nitro, $CF_3$, $CHF_2$, $CH_2F$, $OCF_3$, $OCHF_2$, $OCH_2F$, azido, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''', OR', aryl, heteroaryl, arylalkyl, heteroarylalkyl, heterocyclyl, and heterocyclylalkyl; or

$R_3$ and $R_4$ can be taken together with the atom to which they are attached to form a 4 to 10 membered heteroaryl or heterocyclic ring, each of which is unsubstituted or substituted with 1 to 5 groups independently selected from oxo, halogen, cyano, nitro, $CF_3$, $CHF_2$, $CH_2F$, $OCF_3$, $OCHF_2$, $OCH_2F$, azido, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''', OR', aryl, heteroaryl, arylalkyl, heteroarylalkyl, heterocyclyl, and heterocyclylalkyl;

$R_5$ is selected from hydrogen or $C_1$-$C_6$ alkyl whereby alkyl may be substituted or unsubstituted with 1 to 5 groups independently selected from oxo, halogen, cyano, nitro, $CF_3$, $CHF_2$, $CH_2F$, $OCF_3$, $OCHF_2$, $OCH_2F$, azido, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''', OR', aryl, heteroaryl, arylalkyl, heteroarylalkyl, heterocyclyl, and heterocyclylalkyl; or

$R_4$ and $R_5$ can be taken together with the atom to which they are attached to form a 4 to 10 membered carbocyclic, heteroaryl or heterocyclic ring, each of which is unsubstituted or substituted with 1 to 5 groups independently selected from oxo, halogen, cyano, nitro, $CF_3$, $CHF_2$, $CH_2F$, $OCF_3$, $OCHF_2$, $OCH_2F$, azido, NR'$SO_2$R'''', $SO_2$NR'R-, C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''', OR', aryl, heteroaryl, arylalkyl, heteroarylalkyl, heterocyclyl, and heterocyclylalkyl;

$R_6$ is selected from trifluoromethyl, $C_1$-$C_{10}$ alkyl, $C_3$-$C_{10}$ cycloalkyl, aryl, arylalkyl, heteroaryl, heteroarylalkyl, heterocyclyl, and heterocyclylalkyl, where each alkyl, cycloalkyl, aryl, heteroaryl and heterocyclyl unsubstituted or substituted with 1 to 5 groups independently selected from oxo, halogen, cyano, nitro, $CF_3$, $CHF_2$, $CH_2F$, $OCF_3$, $OCHF_2$, $OCH_2F$, azido, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''', OR', aryl, heteroaryl, arylalkyl, hetero-arylalkyl, heterocyclyl, and heterocyclylalkyl;

W is selected from heteroaryl containing 1-4 heteroatoms or heterocyclyl containing 1-4 heteroatoms each of which is unsubstituted or substituted by 1 to 5 substituents $ZR_{15}$; or W is $-C(O)OR_{15}$, $-C(O)NR_4R_{15}$, $-C(O)NR_4OR_{15}$, -C(O)($C_3$-$C_{10}$ cycloalkyl), -C(O)($C_2$-$C_{10}$ alkyl), -C(O)(aryl), -C(O)(heteroaryl), -C(O)(heterocyclyl), $-S(O)_jNR_4R_{15}$, $-S(O)_jNR_4OR_{15}$, $-S(O)_jNR_4C(O)R_{15}$, or $-C(O)NR_4S(O)_jR_6$, whereby $R_4$ and $R_{15}$ are as defined herein or may form together a 3 to 7 membered ring with 1 or 2 N atoms and optionally an O atom,

Z is a bond, $NR_{16}$, O, $NR_{16}SO_2$ or S,

$R_{15}$ is independently selected from hydrogen, trifluoromethyl, $C_1$-$C_{10}$ alkyl, $C_2$-$C_{10}$ alkenyl, $C_2$-$C_{10}$ alkynyl, $C_3$-$C_{10}$ cycloalkyl, $C_3$-$C_{10}$ cycloalkylalkyl, aryl, arylalkyl, heteroaryl, heteroarylalkyl, heterocyclyl, and heterocyclylalkyl, where each alkyl, alkenyl, alkynyl, cycloalkyl, aryl, heteroaryl and heterocyclyl is unsubstituted or substituted with 1 to 5 substituents selected from $OR_3$ or NR'R'' wherein $R_3$ is selected from hydrogen, $C_1$-$C_4$ alkyl or $C_2$-$C_4$ alkenyl, $C_4$-$C_6$ cycloalkylalkyl, and where R' and R'' are independently hydrogen or $C_1$-$C_4$ alkyl, or R' and R'' may form a 3 to 7 membered ring with 1 or 2 N atoms and optionally an O atom;

$R_{16}$ is selected from hydrogen or $C_1$-$C_{10}$ alkyl, or $R_{15}$ and $R_{16}$ form together a 4 to 10 membered cyclic ring with 1 or 2 N atoms and optionally an O atom, said ring being unsubstituted or substituted by 1 or 2 alkyl amino, amino, hydroxy or O-alkyl;

R' is selected from hydrogen, $C_1$-$C_4$ alkyl, $C_2$-$C_4$ alkenyl, aryl and arylalkyl;

R" is selected from hydrogen, $C_1$-$C_4$ alkyl, $C_2$-$C_4$ alkenyl, aryl and arylalkyl;
R'" is selected from hydrogen, $C_1$-$C_4$ alkyl, $C_2$-$C_4$ alkenyl, aryl and arylalkyl;
R"" is selected from $C_1$-$C_4$ alkyl, $C_2$-$C_4$ alkenyl, aryl and arylalkyl;
or any two of R', R", R'" or R"" can be taken together with the atom to which they are attached to form a 4 to 10 membered carbocyclic, heteroaryl or heterocyclic ring, each of which is optionally substituted with one to three groups independently selected from halogen, cyano; nitro, $CF_3$, $CHF_2$, $CH_2F$, $OCF_3$, $OCHF_2$, $OCH_2F$, azido, aryl, heteroaryl, arylalkyl, heteroarylalkyl, heterocyclyl, and hetero-cyclylalkyl, preferably halogen, cyano; nitro, trifluoromethyl, difluoromethoxy, trifluoromethoxy and azido;
m is 0, 1, 2, 3, 4 or 5 ;and
j is 1 or 2.

**2.** The compound of Formula (I) according to claim 1 wherein

$R_1$, $R_2$, $R_9$, $R_{11}$ are selected independently from hydrogen, halo, $C_1$-$C_4$ alkyl, $C_3$-$C_4$ cycloalkyl, $C_2$-$C_4$ alkenyl, $C_2$-$C_4$ alkynyl, cyano, nitro, $OR_3$ or $NR_3R_4$ where each alkyl, alkenyl, alkynyl, cycloalkyl is optionally substituted with one to five halogens;
$R_{10}$ is selected from hydrogen, $-OR_3$, $-NR_4C(O)R_3$, $-C(O)NR_3R_4$, $-NR_3R_4$;
L is selected from a bond or $C_1$-$C_5$ alkyl;
$R_{12}$ is selected independently from hydrogen, halo, $C_1$-$C_{10}$ alkyl, $C_3$-$C_{10}$ cycloalkyl, $C_2$-$C_{10}$ alkenyl, $C_2$-$C_{10}$ alkynyl, cyano, nitro, azido; $NR_4SO_2R_6$; $SO_2NR_3R_4$; $SO_2R_6$; $C(O)NR_3R_4$; $-S(O)_jNR_4C(O)R_3$, $-C(O)NR_4S(O)_jR_6$, $OR_3$, $NR_3R_4$ or $-S(C_1$-$C_2$ alkyl) substituted with 1 to 5 F, where each alkyl, alkenyl, alkynyl, cycloalkyl, aryl, heteroaryl and heterocyclyl is unsubstituted or substituted with 1 to 5 groups independently selected from oxo, halogen, cyano, nitro, $CF_3$, $CHF_2$, $CH_2F$, $OCF_3$, $OCHF_2$, $OCH_2F$, $SCF_3$, $SCHF_2$, $SCH_2F$, azido, $NR_4SO_2R_6$, $SO_2NR_3R_4$, $C(O)R_3$, $C(O)OR_3$, $OC(O)R_3$, $NR_4C(O)OR_6$, $NR_4C(O)R_3$, $C(O)NR_3R_4$, $NR_3R_4$, $NR_5C(O)NR_3R_4$, $NR_5C(NCN)NR_3R_4$, $OR_3$, aryl, heteroaryl, arylalkyl, hetero-arylalkyl, heterocyclyl, and heterocyclylalkyl;
$R_{13}$ and $R_{14}$ are selected independently from H, F, Cl and $C_1$-$C_4$ alkyl, $C_3$-$C_4$ cycloalkyl, $C_2$-$C_4$ alkenyl, $C_2$-$C_4$ alkynyl where each alkyl, alkenyl, cycloalkyl, alkynyl is optionally further substituted with one to five halogens;
W is selected from heteroaryl containing 1-4 heteroatoms, heterocyclyl containing 1-4 heteroatoms each of which is unsubstituted or substituted by 1 to 3 substituents $ZR_{15}$, or W is $-C(O)OR_{15}$, $-C(O)NR_4R_{15}$, $-C(O)NR_4OR_{15}$, $-C(O)(C_3$-$C_{10}$ cycloalkyl), $-C(O)(C_2$-$C_{10}$ alkyl), $-S(O)_jNR_4C(O)R_{15}$, $-C(O)NR_4S(O)_jR_6$, $S(O)_jNR_4R_{15}$ or $S(O)_jNR_4OR_{15}$;
Z is selected from $NR_{16}$, $NR_{16}SO_2$ or O;
$R_{15}$ is selected from hydrogen, $C_1$-$C_4$ alkyl, $C_2$-$C_4$ alkenyl, $C_4$-$C_6$ cycloalkylalkyl, where alkyl or alkenyl may be further substituted by 1 or 2 of OH, O-$C_1$-$C_4$ alkyl or NR'R";
$R_{16}$ is selected from hydrogen or $C_1$-$C_4$ alkyl;
R' and R" are each independently selected from hydrogen, $C_1$-$C_4$ alkyl, $C_2$-$C_4$ alkenyl, aryl and arylalkyl.

**3.** The compound of Formula (I) according to claim 1 or 2 wherein

$R_1$ is selected independently from H and F;
$R_2$ is selected independently from F, Cl, Me, where the methyl group is optionally substituted with one to three fluorines;
$R_9$ is selected independently from H, F, Cl;
$R_{10}$ is selected from hydrogen, $-OR_3$, $-NR_3R_4$, wherein $R_3$ and $R_4$ are independently $C_1$-$C_6$ alkyl, optionally substituted by 1 or 2 alkyl amino or O-alkyl, or $R_3$ and $R_4$ form together a cyclic ring with 1 or 2 N atoms and optionally an O atom, said ring being optionally substituted by 1 or 2 alkyl amino or O-alkyl
L is selected from a bond, methylene, ethylene, n-propylene or n-butylene;
$R_{11}$ is selected independently from H, F, Cl, Br, Me, OMe, where the methyl groups are optionally substituted with one to three fluorines;
$R_{12}$ is selected independently from H, F, Cl, Br, I, nitro, methyl, ethyl, n-propyl, i-propyl, cyclopropyl, $SCF_3$, $SCHF_2$, $SCH_2F$, $SO_2NR_3R_4$ or $C(O)NR_3R_4$ or OMe, where the methyl groups are optionally substituted with one to three fluorines, wherein $R_3$ and $R_4$ are independently $C_1$-$C_6$ alkyl, optionally substituted by 1 or 2 alkyl amino or O-alkyl, or $R_3$ and $R_4$ form together a cyclic ring with 1 or 2 N atoms and optionally an O atom, said ring being optionally substituted by 1 or 2 alkyl amino or O-alkyl;
$R_{13}$ is selected independently from H and F;
$R_{14}$ is selected independently from H and F;
W is selected from $-C(O)NR_4OR_{15}$ or $SO_2NR_4OR_{15}$;

or W is

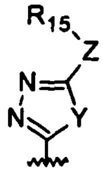

wherein

Z is $NR_{16}$;
$R_{15}$ is $C_2$-$C_4$ alkyl or $C_2$-$C_4$ alkenyl optionally substituted with 1 to 3 substituents OH, O-Me, $NH_2$, $N(methyl)_2$, NHmethyl, NHethyl or $N(ethyl)_2$;
$R_{16}$ is hydrogen or $C_1$-$C_4$ alkyl, or $R_{16}$ and $R_{15}$ form together a 4 to 10 membered ring with 1 or 2 N atoms and optionally an O atom, said ring being optionally substituted by 1 or 2 alkyl amino, amino, hydroxy or O-alkyl.
Y is O, S or NR'.

**4.** The compound of Formula (I) according to any of claims 1 to 3 wherein

W is selected from $-C(O)NR_4OR_{15}$ or $SO_2NR_4OR_{15}$,

or W is

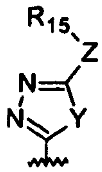

wherein

$R_4$ is hydrogen;
Z is NH,
$R_{15}$ is selected from $C_1$-$C_4$ alkyl or $C_2$-$C_4$ alkenyl that may be further substituted by 1 or 2 of OH, O-$C_1$-$C_4$ alkyl or NR'R",
R' and R" are independently hydrogen, methyl or ethyl; and
Y is O.

**5.** The compound of Formula (I) according to any of claims 1 to 4 wherein $LR_{10}$ are together methyl or hydrogen.

**6.** The compound of any of claims 1 to 5 for use as a medicament.

**7.** The compound according to any of claims 1 to 5, for use in the treatment of hyperproliferative diseases or disorders mediated by aberrant proliferation, including cancer.

**8.** The compound of any of claims 1 to 5 for use in the treatment of hyper-proliferative diseases related to the hyperactivity of MEK as well as diseases modulated by the MEK cascade in mammals.

**9.** The compound according to claim 8 for the treatment of diseases selected from the group consisting of cancer, inflammation, pancreatitis or kidney disease, pain, benign hyperplasia of the skin, restenosis, prostate, diseases related to vasculogenesis or angiogenesis, tumor angiogenesis, skin diseases selected from psoriasis, eczema, and sclerodema, diabetes, diabetic retinopathy, retinopathy of prematurity, age-related macular degeneration, hemangioma, glioma, melanoma and Kaposi's sarcoma.

**10.** The compound according to claim 8 or 9 for the treatment of cancer or inflammation.

**11.** The compound according to any of claims 8 to 10 for the treatment of cancer selected from the group consisting of ovarian, breast, lung, pancreatic, prostate, colon and epidermoid cancer; or inflammation selected from the group consisting of rheumatoid arthritis, inflammatory bowel disease, atherosclerosis:

**12.** Use of a compound according to any of claims 1 to 5 in the preparation of a medicament in the treatment of hyperproliferative diseases or disorders mediated by aberrant proliferation, including cancer, hyperproliferative diseases related to the hyperactivity of MEK as well as diseases modulated by the MEK cascade in mammals.

**13.** A pharmaceutical composition which comprises a compound of any of claims 1 to 5 and a pharmaceutically acceptable carrier.

**Patentansprüche**

**1.** Verbindung der Formel (I)

Formel (I) 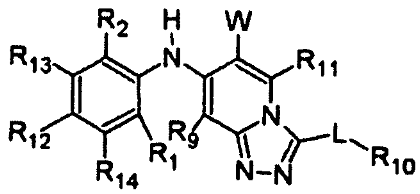

ein pharmazeutisch verträgliches Salz oder Solvat davon, wobei:

$R_1$, $R_2$, $R_9$, $R_{11}$, $R_{12}$, $R_{13}$ und $R_{14}$ sind unabhängig voneinander ausgewählt aus Wasserstoff, Halogen, Cyano, Nitro, Azido, $-OR_3$, $-C(O)R_3$, $-C(O)OR_3$, $-NR_4C(O)OR_6$, $-OC(O)R_3$, $-NR_4S(O)_jR_6$, $-S(O)_jNR_3R_4$, $-S(O)_jNR_4C(O)R_3$, $-C(O)NR_4S(O)_jR_6$, $S(O)_jR_6$, $-NR_4C(O)R_3$, $-C(O)NR_3R_4$, $-NR_5C(O)NR_3R_4$, $-NR_5C(NCN)NR_3R_4$, $-NR_3R_4$ und $C_1$-$C_{10}$-Alkyl, $C_2$-$C_{10}$-Alkenyl, $C_2$-$C_{10}$-Alkinyl, $C_3$-$C_{10}$-Cycloalkyl, $C_3$-$C_{10}$-Cycloalkylalkyl, $-S(O)_j(C_1$-$C_6$-Alkyl), $-S(O)_j(CR_4R_5)_m$-Aryl, Aryl, Arylalkyl, Heteroaryl, Heteroarylalkyl, Heterocyclyl, Heterocyclylalkyl, $-O(CR_4R_5)_m$-Aryl, $-NR_4(CR_4R_5)_m$-Aryl, $-O(CR_4R_5)_m$-Heteroaryl, $-NR_4(CR_4R_5)_m$-Heteroaryl, $-O(CR_4R_5)_m$-Heterocyclyl, $-NR_4(CR_4R_5)_m$-Heterocyclyl und $-S(C_1$-$C_2$-Alkyl), substituiert mit 1 bis 5 F, wobei jedes Alkyl, Alkenyl, Alkinyl, Cycloalkyl, Aryl, Heteroaryl und Heterocyclyl unsubstituiert oder mit 1 bis 5 Gruppen substituiert ist, die unabhängig voneinander ausgewählt sind aus Oxo, Halogen, Cyano, Nitro, $CF_3$, $CHF_2$, $CH_2F$, $OCF_3$, $OCHF_2$, $OCH_2F$, $SCF_3$, $SCHF_2$, $SCH_2F$, Azido, $NR_4SO_2R_6$, $SO_2NR_3R_4$, $C(O)R_3$, $C(O)OR_3$, $OC(O)R_3$, $NR_4C(O)OR_6$, $NR_4C(O)R_3$, $C(O)NR_3R_4$, $NR_3R_4$, $NR_5C(O)NR_3R_4$, $NR_5C(NCN)NR_3R_4$, $OR_3$, Aryl, Heteroaryl, Arylalkyl, Heteroarylalkyl, Heterocyclyl und Heterocyclylalkyl;

$R_{10}$ ist ausgewählt aus Wasserstoff, $-OR_3$, $-C(O)R_3$, $-C(O)OR_3$, $-NR_4C(O)OR_6$, $-OC(O)R_3$, $-NR_4S(O)_jR_6$, $-S(O)_jNR_3R_4$, $-S(O)_jNR_4C(O)R_3$, $-C(O)NR_4S(O)_jR_6$, $S(O)_jR_6$, $-NR_4C(O)R_3$, $-C(O)NR_3R_4$, $-NR_5C(O)NR_3R_4$, $-NR_5C(NCN)NR_3R_4$, $-NR_3R_4$, $-S(O)_j(C_1$-$C_6$-Alkyl), $-S(O)_j(CR_4R_5)_m$-Aryl, $-O(CR_4R_5)_m$-Aryl, $-NR_4(CR_4R_5)_m$-Aryl, $-O(CR_4R_5)_m$-Heteroaryl, $-NR_4(CR_4R_5)_m$-Heteroaryl, $-O(CR_4R_5)_m$-Heterocyclyl, $-NR_4(CR_4R_5)_m$-Heterocyclyl und $-S(C_1$-$C_2$-Alkyl), substituiert mit 1 bis 5 F, wobei jedes Aryl, Heteroaryl und Heterocyclyl unsubstituiert oder mit 1 bis 5 Gruppen substituiert ist, die unabhängig voneinander ausgewählt sind aus Oxo, Halogen, Cyano, Nitro, $CF_3$, $CHF_2$, $CH_2F$, $OCF_3$, $OCHF_2$, $OCH_2F$, $SCF_3$, $SCHF_2$, $SCH_2F$, Azido, $NR_4SO_2R_6$, $SO_2NR_3R_4$, $C(O)R_3$, $C(O)OR_3$, $OC(O)R_3$, $NR_4C(O)OR_6$, $NR_4C(O)R_3$, $C(O)NR_3R_4$, $NR_3R_4$, $NR_5C(O)NR_3R_4$, $NR_5C(NCN)NR_3R_4$, $OR_3$, Aryl, Heteroaryl, Arylalkyl, Heteroarylalkyl, Heterocyclyl und Heterocyclylalkyl;

L ist ausgewählt aus einer Bindung, $C_1$-$C_{10}$-Alkyl, $C_2$-$C_{10}$-Alkenyl, $C_2$-$C_{10}$-Alkinyl, $C_3$-$C_{10}$-Cycloalkyl, $C_3$-$C_{10}$-Cycloalkylalkyl, Aryl, Arylalkyl, Heteroaryl, Heteroarylalkyl, Heterocyclyl, Heterocyclylalkyl, wobei jedes Alkyl, Alkenyl, Alkinyl, Cycloalkyl, Aryl, Heteroaryl und Heterocyclyl unsubstituiert oder mit 1 bis 5 Gruppen substituiert ist, die unabhängig voneinander ausgewählt sind aus Oxo, Halogen, Cyano, Nitro, $CF_3$, $CHF_2$, $CH_2F$, $OCF_3$, $OCHF_2$, $OCH_2F$, $SCF_3$, $SCHF_2$, $SCH_2F$, Azido, $NR_4SO_2R_6$, $SO_2NR_3R_4$, $C(O)R_3$, $C(O)OR_3$, $OC(O)R_3$, $NR_4C(O)OR_6$, $NR_4C(O)R_3$, $C(O)NR_3R_4$, $NR_3R_4$, $NR_5C(O)NR_3R_4$, $NR_5C(NCN)NR_3R_4$, $OR_3$, Aryl, Heteroaryl, Arylalkyl, Heteroarylalkyl, Heterocyclyl und Heterocyclylalkyl;

$R_3$ ist ausgewählt aus Wasserstoff, Trifluormethyl, $C_1$-$C_{10}$-Alkyl, $C_2$-$C_{10}$-Alkenyl, $C_2$-$C_{10}$-Alkinyl, $C_3$-$C_{10}$-Cycloalkyl, $C_3$-$C_{10}$-Cycloalkylalkyl, Aryl, Arylalkyl, Heteroaryl, Heteroarylalkyl, Heterocyclyl und Heterocyclylalkyl,

wobei jedes Alkyl, Alkenyl, Alkinyl, Cycloalkyl, Aryl, Heteroaryl und Heterocyclyl unsubstituiert oder mit 1 bis 5 Gruppen substituiert ist, die unabhängig voneinander ausgewählt sind aus Oxo, Halogen, Cyano, Nitro, $CF_3$, $CHF_2$, $CH_2F$, $OCF_3$, $OCHF_2$, $OCH_2F$, Azido, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''', OR', Aryl, Heteroaryl, Arylalkyl, Heteroarylalkyl, Heterocyclyl und Heterocyclylalkyl;

$R_4$ ist ausgewählt aus Wasserstoff oder $C_1$-$C_6$-Alkyl, wobei Alkyl unsubstituiert oder mit 1 bis 5 Gruppen substituiert sein kann, die unabhängig voneinander ausgewählt sind aus Oxo, Halogen, Cyano, Nitro, $CF_3$, $CHF_2$, $CH_2F$, $OCF_3$, $OCHF_2$, $OCH_2F$, Azido, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''', OR', Aryl, Heteroaryl, Arylalkyl, Heteroarylalkyl, Heterocyclyl und Heterocyclylalkyl; oder

$R_3$ und $R_3$ können mit dem Atom, an das sie gebunden sind, zusammengefasst werden, um einen 4- bis 10-gliedrigen Heteroaryl- oder heterocyclischen Ring zu bilden, von denen jeder unsubstituiert oder mit 1 bis 5 Gruppen substituiert ist, die unabhängig voneinander ausgewählt sind aus Oxo, Halogen, Cyano, Nitro, $CF_3$, $CHF_2$, $CH_2F$, $OCF_3$, $OCHF_2$, $OCH_2F$, Azido, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''', OR', Aryl, Heteroaryl, Arylalkyl, Heteroarylalkyl, Heterocyclyl und Heterocyclylalkyl;

$R_5$ ist ausgewählt aus Wasserstoff oder $C_1$-$C_6$-Alkyl, wobei Alkyl unsubstituiert oder mit 1 bis 5 Gruppen substituiert sein kann, die unabhängig voneinander ausgewählt sind aus Oxo, Halogen, Cyano, Nitro, $CF_3$, $CHF_2$, $CH_2F$, $OCF_3$, $OCHF_2$, $OCH_2F$, Azido, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''', OR', Aryl, Heteroaryl, Arylalkyl, Heteroarylalkyl, Heterocyclyl und Heterocyclylalkyl; oder

$R_4$ und $R_5$ können mit dem Atom, an das sie gebunden sind, zusammengefasst werden, um einen 4- bis 10-gliedrigen carbocyclischen, Heteroaryl- oder heterocyclischen Ring zu bilden, von denen jeder unsubstituiert oder mit 1 bis 5 Gruppen substituiert ist, die unabhängig voneinander ausgewählt sind aus Oxo, Halogen, Cyano, Nitro, $CF_3$, $CHF_2$, $CH_2F$, $OCF_3$, $OCHF_2$, $OCH_2F$, Azido, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''', OR', Aryl, Heteroaryl, Arylalkyl, Heteroarylalkyl, Heterocyclyl und Heterocyclylalkyl;

$R_6$ ist ausgewählt aus Trifluormethyl, $C_1$-$C_{10}$-Alkyl, $C_3$-$C_{10}$-Cycloalkyl, Aryl, Arylalkyl, Heteroaryl, Heteroarylalkyl, Heterocyclyl und Heterocyclylalkyl, wobei jedes Alkyl, Cycloalkyl, Aryl, Heteroaryl und Heterocyclyl unsubstituiert oder mit 1 bis 5 Gruppen substituiert ist, die unabhängig voneinander ausgewählt sind aus Oxo, Halogen, Cyano, Nitro, $CF_3$, $CHF_2$, $CH_2F$, $OCF_3$, $OCHF_2$, $OCH_2F$, Azido, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''', OR', Aryl, Heteroaryl, Arylalkyl, Heteroarylalkyl, Heterocyclyl und Heterocyclylalkyl;

W ist ausgewählt aus Heteroaryl, enthaltend 1-4 Heteroatome, oder Heterocyclyl, enthaltend 1-4 Heteroatome, von denen jedes unsubstituiert oder durch 1 bis 5 Substituenten $ZR_{15}$ substituiert ist; oder W ist -C(O)$OR_{15}$, -C(O)$NR_4R_{15}$, -C(O)$NR_4OR_{15}$, -C(O)($C_3$-$C_{10}$-Cycloalkyl), -C(O)($C_2$-$C_{10}$-Alkyl), -C(O)(Aryl), -C(O)(Heteroaryl), -C(O)(Heterocyclyl), -$S(O)_jNR_4R_{15}$, -$S(O)_jNR_4OR_{15}$, -$S(O)_jNR_4C(O)R_{15}$ oder -C(O)$NR_4S(O)_jR_6$, wobei $R_4$ und $R_{15}$ wie hier definiert sind oder zusammen einen 3- bis 7-gliedrigen Ring mit 1 oder 2 N-Atomen und gegebenenfalls einem O-Atom bilden können,

Z ist eine Bindung, $NR_{16}$, O, $NR_{16}SO_2$ oder S,

$R_{15}$ ist unabhängig ausgewählt aus Wasserstoff, Trifluormethyl, $C_1$-$C_{10}$-Alkyl, $C_2$-$C_{10}$-Alkenyl, $C_2$-$C_{10}$-Alkinyl, $C_3$-$C_{10}$-Cycloalkyl, $C_3$-$C_{10}$-Cycloalkylalkyl, Aryl, Arylalkyl, Heteroaryl, Heteroarylalkyl, Heterocyclyl und Heterocyclylalkyl, wobei jedes Alkyl, Alkenyl, Alkinyl, Cycloalkyl, Aryl, Heteroaryl und Heterocyclyl unsubstituiert oder mit 1 bis 5 Substituenten substituiert ist, die ausgewählt sind aus $OR_3$ oder NR'R'', wobei $R_3$ ausgewählt ist aus Wasserstoff, $C_1$-$C_4$-Alkyl oder $C_2$-$C_4$-Alkenyl, $C_4$-$C_6$-Cycloalkylalkyl, und wobei R' und R'' unabhängig voneinander Wasserstoff oder $C_1$-$C_4$-Alkyl sind oder R' und R'' einen 3- bis 7-gliedrigen Ring mit 1 oder 2 N-Atomen und gegebenenfalls einem O-Atom bilden können;

$R_{16}$ ist ausgewählt aus Wasserstoff oder $C_1$-$C_{10}$-Alkyl oder $R_{15}$ und $R_{16}$ bilden zusammen einen 4- bis 10-gliedrigen cyclischen Ring mit 1 oder 2 N-Atomen und gegebenenfalls einem O-Atom, wobei der Ring unsubstituiert oder durch 1 oder 2 Alkylamino, Amino, Hydroxy oder O-Alkyl substituiert ist;

R' ist ausgewählt aus Wasserstoff, $C_1$-$C_4$-Alkyl, $C_2$-$C_4$-Alkenyl, Aryl und Arylalkyl;

R'' ist ausgewählt aus Wasserstoff, $C_1$-$C_4$-Alkyl, $C_2$-$C_4$-Alkenyl, Aryl und Arylalkyl;

R''' ist ausgewählt aus Wasserstoff, $C_1$-$C_4$-Alkyl, $C_2$-$C_4$-Alkenyl, Aryl und Arylalkyl;

R'''' ist ausgewählt aus $C_1$-$C_4$-Alkyl, $C_2$-$C_4$-Alkenyl, Aryl und Arylalkyl;

oder beliebige zwei von R', R'', R''' oder R'''' können mit dem Atom, an das sie gebunden sind, zusammengefasst werden, um einen 4- bis 10-gliedrigen carbocyclischen, Heteroaryl- oder heterocyclischen Ring zu bilden, von denen jeder gegebenenfalls mit einer bis drei Gruppen substituiert ist, die unabhängig voneinander ausgewählt sind aus Halogen, Cyano, Nitro, $CF_3$, $CHF_2$, $CH_2F$, $OCF_3$, $OCHF_2$, $OCH_2F$, Azido, Aryl, Heteroaryl, Arylalkyl,

Heteroarylalkyl, Heterocyclyl und Heterocyclylalkyl, vorzugsweise Halogen, Cyano, Nitro, Trifluormethyl, Difluormethoxy, Trifluormethoxy und Azido;
m ist 0, 1, 2, 3, 4 oder 5; und
j ist 1 oder 2.

**2.** Verbindung der Formel (I) nach Anspruch 1, wobei

$R_1$, $R_2$, $R_9$, $R_{11}$ sind unabhängig voneinander ausgewählt aus Wasserstoff, Halogen, $C_1$-$C_4$-Alkyl, $C_3$-$C_4$-Cycloalkyl, $C_2$-$C_4$-Alkenyl, $C_2$-$C_4$-Alkinyl, Cyano, Nitro, $OR_3$ oder $NR_3R_4$, wobei jedes Alkyl, Alkenyl, Alkinyl, Cycloalkyl gegebenenfalls mit einem bis fünf Halogenen substituiert ist;
$R_{10}$ ist ausgewählt aus Wasserstoff, $-OR_3$, $-NR_4C(O)R_3$, $-C(O)NR_3R_4$, $-NR_3R_4$;
L ist ausgewählt aus einer Bindung oder $C_1$-$C_5$-Alkyl;
$R_{12}$ ist unabhängig ausgewählt aus Wasserstoff, Halogen, $C_1$-$C_{10}$-Alkyl, $C_3$-$C_{10}$-Cycloalkyl, $C_2$-$C_{10}$-Alkenyl, $C_2$-$C_{10}$-Alkinyl, Cyano, Nitro, Azido, $NR_4SO_2R_6$, $SO_2NR_3R_4$, $SO_2R_6$, $C(O)NR_3R_4$, $-S(O)_jNR_4C(O)R_3$, $-C(O)NR_4S(O)_jR_6$, $OR_3$, $NR_3R_4$ oder $-S(C_1$-$C_2$-Alkyl), substituiert mit 1 bis 5 F, wobei jedes Alkyl, Alkenyl, Alkinyl, Cycloalkyl, Aryl, Heteroaryl und Heterocyclyl unsubstituiert oder mit 1 bis 5 Gruppen substituiert ist, die unabhängig voneinander ausgewählt sind aus Oxo, Halogen, Cyano, Nitro, $CF_3$, $CHF_2$, $CH_2F$, $OCF_3$, $OCHF_2$, $OCH_2F$, $SCF_3$, $SCHF_2$, $SCH_2F$, Azido, $NR_4SO_2R_6$, $SO_2NR_3R_4$, $C(O)R_3$, $C(O)OR_3$, $OC(O)R_3$, $NR_4C(O)OR_6$, $NR_4C(O)R_3$, $C(O)NR_3R_4$, $NR_3R_4$, $NR_5C(O)NR_3R_4$, $NR_5C(NCN)NR_3R_4$, $OR_3$, Aryl, Heteroaryl, Arylalkyl, Heteroarylalkyl, Heterocyclyl und Heterocyclylalkyl;
$R_{13}$ und $R_{14}$ sind unabhängig voneinander ausgewählt aus H, F, Cl und $C_1$-$C_4$-Alkyl, $C_3$-$C_4$-Cycloalkyl, $C_2$-$C_4$-Alkenyl, $C_2$-$C_4$-Alkinyl, wobei jedes Alkyl, Alkenyl, Cycloalkyl, Alkinyl gegebenenfalls mit einem bis fünf Halogenen weiter substituiert ist;
W ist ausgewählt aus Heteroaryl, enthaltend 1-4 Heteroatome, Heterocyclyl, enthaltend 1-4 Heteroatome, von denen jedes unsubstituiert oder durch 1 bis 3 Substituenten $\mathbf{ZR_{15}}$ substituiert ist, oder W ist $-C(O)OR_{15}$, $-C(O)NR_4R_{15}$, $-C(O)NR_4OR_{15}$, $-C(O)(C_3$-$C_{10}$-Cycloalkyl), $-C(O)(C_2$-$C_{10}$-Alkyl), $-S(O)_jNR_4C(O)R_{15}$, $-C(O)NR_4S(O)_jR_6$, $S(O)_jNR_4R_{15}$ oder $S(O)_jNR_4OR_{15}$;
Z ist ausgewählt aus $NR_{16}$, $NR_{16}SO_2$ oder O;
$R_{15}$ ist ausgewählt aus Wasserstoff, $C_1$-$C_4$-Alkyl, $C_2$-$C_4$-Alkenyl, $C_4$-$C_6$-Cycloalkylalkyl, wobei Alkyl oder Alkenyl durch 1 oder 2 von OH, O-$C_1$-$C_4$-Alkyl oder NR'R" weiter substituiert sein können;
$R_{16}$ ist ausgewählt aus Wasserstoff oder $C_1$-$C_4$-Alkyl;
R' und R" sind jeweils unabhängig voneinander ausgewählt aus Wasserstoff, $C_1$-$C_4$-Alkyl, $C_2$-$C_4$-Alkenyl, Aryl und Arylalkyl.

**3.** Verbindung der Formel (I) nach Anspruch 1 oder 2, wobei

$R_1$ ist unabhängig ausgewählt aus H und F;
$R_2$ ist unabhängig ausgewählt aus F, Cl, Me, wobei die Methylgruppe gegebenenfalls mit einem bis drei Fluoratomen substituiert ist;
$R_9$ ist unabhängig ausgewählt aus H, F, Cl;
$R_{10}$ ist ausgewählt aus Wasserstoff, $-OR_3$, $-NR_3R_4$, wobei $R_3$ und $R_4$ unabhängig voneinander $C_1$-$C_6$-Alkyl, gegebenenfalls substituiert durch 1 oder 2 Alkylamino oder O-Alkyl, sind oder $R_3$ und $R_4$ zusammen einen cyclischen Ring mit 1 oder 2 N-Atomen und gegebenenfalls einem O-Atom bilden, wobei der Ring gegebenenfalls durch 1 oder 2 Alkylamino oder O-Alkyl substituiert ist;
L ist ausgewählt aus einer Bindung, Methylen, Ethylen, n-Propylen oder n-Butylen;
$R_{11}$ ist unabhängig ausgewählt aus H, F, Cl, Br, Me, OMe, wobei die Methylgruppen gegebenenfalls mit einem bis drei Fluoratomen substituiert sind;
$R_{12}$ ist unabhängig ausgewählt aus H, F, Cl, Br, I, Nitro, Methyl, Ethyl, n-Propyl, i-Propyl, Cyclopropyl, $SCF_3$, $SCHF_2$, $SCH_2F$, $SO_2NR_3R_4$ oder $C(O)NR_3R_4$ oder OMe, wobei die Methylgruppen gegebenenfalls mit einem bis drei Fluoratomen substituiert sind, wobei $R_3$ und $R_4$ unabhängig voneinander $C_1$-$C_6$-Alkyl, gegebenenfalls substituiert durch 1 oder 2 Alkylamino oder O-Alkyl, sind oder $R_3$ und $R_4$ zusammen einen cyclischen Ring mit 1 oder 2 N-Atomen und gegebenenfalls einem O-Atom bilden, wobei der Ring gegebenenfalls durch 1 oder 2 Alkylamino oder O-Alkyl substituiert ist;
$R_{13}$ ist unabhängig ausgewählt aus H und F;
$R_{14}$ ist unabhängig ausgewählt aus H und F;
W ist ausgewählt aus $-C(O)NR_4OR_{15}$ oder $SO_2NR_4OR_{15}$;

oder W ist

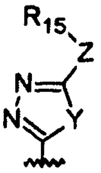

wobei

Z ist $NR_{16}$;
$R_{15}$ ist $C_1$-$C_4$-Alkyl oder $C_2$-$C_4$-Alkenyl, gegebenenfalls substituiert mit 1 bis 3 Substituenten OH, O-Me, $NH_2$, $N(Methyl)_2$, NHMethyl, NHEthyl oder $N(Ethyl)_2$;
$R_{16}$ ist Wasserstoff oder $C_1$-$C_4$-Alkyl oder $R_{16}$ und $R_{15}$ bilden zusammen einen 4- bis 10-gliedrigen Ring mit 1 oder 2 N-Atomen und gegebenenfalls einem O-Atom, wobei der Ring gegebenenfalls durch 1 oder 2 Alkylamino, Amino, Hydroxy oder O-Alkyl substituiert ist;
Y ist O, S oder NR'.

**4.** Verbindung der Formel (I) nach einem der Ansprüche 1 bis 3, wobei

W ist ausgewählt aus $-C(O)NR_4OR_{15}$ oder $SO_2NR_4OR_{15}$,

oder W ist

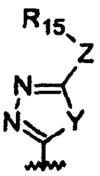

wobei

$R_4$ ist Wasserstoff;
Z ist NH,
$R_{15}$ ist ausgewählt aus $C_1$-$C_4$-Alkyl oder $C_2$-$C_4$-Alkenyl, welches durch 1 oder 2 von OH, O-$C_1$-$C_4$-Alkyl oder NR'R" weiter substituiert sein kann,
R' und R" sind unabhängig voneinander Wasserstoff, Methyl oder Ethyl; und
Y ist O.

**5.** Verbindung der Formel (I) nach einem der Ansprüche 1 bis 4, wobei $LR_{10}$ zusammen Methyl oder Wasserstoff sind.

**6.** Verbindung nach einem der Ansprüche 1 bis 5 zur Verwendung als ein Medikament.

**7.** Verbindung nach einem der Ansprüche 1 bis 5 zur Verwendung bei der Behandlung von hyperproliferativen Erkrankungen oder Störungen, die durch eine aberrante Proliferation vermittelt werden, einschließlich Krebs.

**8.** Verbindung nach einem der Ansprüche 1 bis 5 zur Verwendung bei der Behandlung von hyperproliferativen Erkrankungen, die mit der Hyperaktivität von MEK verbunden sind, sowie Erkrankungen, die durch die MEK-Kaskade moduliert werden, in Säugern.

**9.** Verbindung nach Anspruch 8 für die Behandlung von Erkrankungen, ausgewählt aus der Gruppe bestehend aus Krebs, einer Entzündung, Pankreatitis oder Nierenerkrankung, Schmerzen, gutartiger Hyperplasie der Haut, Restenose, Prostata, Erkrankungen, die mit der Vaskulogenese oder Angiogenese zusammenhängen, Tumorangiogenese, Hauterkrankungen, ausgewählt aus Psoriasis, Ekzem und Sklerodermie, Diabetes, diabetischer Retinopathie, Frühgeborenenretinopathie, altersbedingter Makuladegeneration, Hämangiom, Gliom, Melanom und Kaposi-Sarkom.

**10.** Verbindung nach Anspruch 8 oder 9 für die Behandlung von Krebs oder einer Entzündung.

**11.** Verbindung nach einem der Ansprüche 8 bis 10 für die Behandlung von Krebs, ausgewählt aus der Gruppe bestehend aus Eierstock-, Brust-, Lungen-, Pankreas-, Prostata-, Dickdarm- und Epidermoidkrebs bzw. Plattenepithelkarzinom; oder einer Entzündung, ausgewählt aus der Gruppe bestehend aus rheumatoider Arthritis, entzündlicher Darmerkrankung, Atherosklerose.

**12.** Verwendung einer Verbindung nach einem der Ansprüche 1 bis 5 bei der Herstellung eines Medikaments für die Behandlung von hyperproliferativen Erkrankungen oder Störungen, die durch eine aberrante Proliferation vermittelt werden, einschließlich Krebs, hyperproliferativen Erkrankungen, die mit der Hyperaktivität von MEK verbunden sind, sowie Erkrankungen, die durch die MEK-Kaskade moduliert werden, in Säugern.

**13.** Pharmazeutische Zusammensetzung, welche eine Verbindung nach einem der Ansprüche 1 bis 5 und einen pharmazeutisch verträglichen Träger umfasst.

## Revendications

**1.** Composé de formule (I) :

Formule (I)

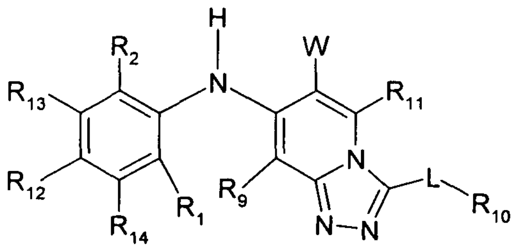

ou sel ou solvat pharmacologiquement admissible d'un tel composé,
dans laquelle formule :

- $R_1$, $R_2$, $R_9$, $R_{11}$, $R_{12}$, $R_{13}$ et $R_{14}$ représentent chacun, indépendamment, une entité choisie parmi les atomes d'hydrogène et d'halogène, les groupes cyano, nitro et azido, les groupes de formule $-OR_3$, $-C(O)R_3$, $-C(O)OR_3$, $-NR_4C(O)OR_6$, $-OC(O)R_3$, $-NR_4S(O)_jR_6$, $-S(O)_jNR_3R_4$, $-S(O)_jNR_4C(O)R_3$, $-C(O)NR_4S(O)_jR_6$, $-S(O)_jR_6$, $-NR_4C(O)R_3$, $-C(O)NR_3R_4$, $-NR_5C(O)NR_3R_4$, $-NR_5C(NCN)NR_3R_4$ ou $-NR_3R_4$, les groupes alkyle en $C_{1-10}$, alcényle en $C_{2-10}$, alcynyle en $C_{2-10}$, cycloalkyle en $C_{3-10}$, (cycloalkyle en $C_{3-10}$)-alkyle, aryle, aryl-alkyle, hétéroaryle, hétéroaryl-alkyle, hétérocyclyle, hétérocyclyl-alkyle, et les groupes symbolisés par $-S(O)_j$(alkyle en $C_{1-6}$), $-S(O)_j-(CR_4R_5)_m$-aryle, $-O(CR_4R_5)_m$-aryle, $-NR_4(CR_4R_5)_m$-aryle, $-O(CR_4R_5)_m$-hétéroaryle, $-NR_4(CR_4R_5)_m$-hétéroaryle, $-O(CR_4R_5)_m$-hétérocyclyle, $-NR_4-(CR_4R_5)_m$-hétérocyclyle et -S-(alkyle en $C_{1-2}$) portant 1 à 5 substituant(s) fluoro, étant entendu que chacun de ces groupes alkyle, alcényle, alcynyle, cycloalkyle, aryle, hétéroaryle et hétérocyclyle ne porte aucun substituant ou porte 1 à 5 substituant(s) choisi(s) indépendamment parmi les substituants oxo, halogéno, cyano, nitro et azido, les substituants représentés par les formules $CF_3$, $CHF_2$, $CH_2F$, $OCF_3$, $OCHF_2$, $OCH_2F$, $SCF_3$, $SCHF_2$, $SCH_2F$, $NR_4SO_2R_6$, $SO_2NR_3R_4$, $C(O)R_3$, $C(O)OR_3$, $OC(O)R_3$, $NR_4C(O)OR_6$, $NR_4C(O)R_3$, $C(O)NR_3R_4$, $NR_3R_4$, $NR_5C(O)NR_3R_4$, $NR_5C(NCN)NR_3R_4$ et $OR_3$, et les substituants aryle, hétéroaryle, aryl-alkyle, hétéroaryl-alkyle, hétérocyclyle et hétérocyclyl-alkyle ;
- $R_{10}$ représente une entité choisie parmi un atome d'hydrogène, les groupes de formule $-OR_3$, $-C(O)R_3$, $-C(O)OR_3$, $-NR_4C(O)OR_6$, $-OC(O)R_3$, $-NR_4S(O)_jR_6$, $-S(O)_jNR_3R_4$, $-S(O)_jNR_4C(O)R_3$, $-C(O)NR_4S(O)_jR_6$, $-S(O)_jR_6$, $-NR_4C(O)R_3$, $-C(O)NR_3R_4$, $-NR_5C(O)NR_3R_4$, $-NR_3R_4$ ou $-NR_5C(NCN)NR_3R_4$, et les groupes symbolisés par $-S(O)_j$(alkyle en $C_{1-6}$), $-S(O)_j-(CR_4R_5)_m$-aryle, $-O(CR_4R_5)_m$-aryle, $-NR_4(CR_4R_5)_m$-aryle, $-O(CR_4R_5)_m$-hétéroaryle, $-NR_4(CR_4R_5)_m$-hétéroaryle, $-O(CR_4R_5)_m$-hétérocyclyle, $-NR_4-(CR_4R_5)_m$-hétérocyclyle et -S-(alkyle en $C_{1-2}$) portant 1 à 5 substituant(s) fluoro, étant entendu que chacun de ces groupes aryle, hétéroaryle et hétérocyclyle ne porte aucun substituant ou porte 1 à 5 substituant(s) choisi(s) indépendamment parmi les substituants oxo, halogéno, cyano, nitro et azido, les substituants représentés par les formules $CF_3$, $CHF_2$, $CH_2F$, $OCF_3$, $OCHF_2$, $OCH_2F$, $SCF_3$, $SCHF_2$, $SCH_2F$, $NR_4SO_2R_6$, $SO_2NR_3R_4$, $C(O)R_3$, $C(O)OR_3$, $OC(O)R_3$, $NR_4C(O)OR_6$, $NR_4C(O)R_3$, $C(O)NR_3R_4$, $NR_3R_4$, $NR_5C(O)NR_3R_4$, $NR_5C(NCN)NR_3R_4$ et $OR_3$, et les substituants

aryle, hétéroaryle, aryl-alkyle, hétéroaryl-alkyle, hétérocyclyle et hétérocyclyl-alkyle ;

- L représente une entité choisie parmi une liaison et les groupes alkyle en $C_{1-10}$, alcényle en $C_{2-10}$, alcynyle en $C_{2-10}$, cycloalkyle en $C_{3-10}$, (cycloalkyle en $C_{3-10}$)-alkyle, aryle, aryl-alkyle, hétéroaryle, hétéroaryl-alkyle, hétérocyclyle et hétérocyclyl-alkyle, étant entendu que chacun de ces groupes alkyle, alcényle, alcynyle, cycloalkyle, aryle, hétéro-aryle et hétérocyclyle ne porte aucun substituant ou porte 1 à 5 substi-tuant(s) choisi(s) indépendamment parmi les substituants oxo, halogé-no, cyano, nitro et azido, les substituants représentés par les formules $CF_3$, $CHF_2$, $CH_2F$, $OCF_3$, $OCHF_2$, $OCH_2F$, $SCF_3$, $SCHF_2$, $SCH_2F$, $NR_4SO_2R_6$, $SO_2NR_3R_4$, $C(O)R_3$, $C(O)OR_3$, $OC(O)R_3$, $NR_4C(O)OR_6$, $NR_4C(O)R_3$, $C(O)NR_3R_4$, $NR_3R_4$, $NR_5C(O)NR_3R_4$, $NR_5C(NCN)NR_3R_4$ et $OR_3$, et les substituants aryle, hétéroaryle, aryl-alkyle, hétéroaryl-alkyle, hétérocyclyle et hétérocyclyl-alkyle ;

- $R_3$ représente une entité choisie parmi un atome d'hydrogène et les groupes trifluorométhyle, alkyle en $C_{1-10}$, alcényle en $C_{2-10}$, alcynyle en $C_{2-10}$, cycloalkyle en $C_{3-10}$, (cycloalkyle en $C_{3-10}$)-alkyle, aryle, aryl-alkyle, hétéroaryle, hétéroaryl-alkyle, hétérocyclyle et hétérocyclyl-alkyle, étant entendu que chacun de ces groupes alkyle, alcényle, alcynyle, cycloalkyle, aryle, hétéroaryle et hétérocyclyle ne porte aucun substituant ou porte 1 à 5 substituant(s) choisi(s) indépendamment parmi les substituants oxo, halogéno, cyano, nitro et azido, les substituants représentés par les formules $CF_3$, $CHF_2$, $CH_2F$, $OCF_3$, $OCHF_2$, $OCH_2F$, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''' et OR', et les substituants aryle, hétéroaryle, aryl-alkyle, hétéroaryl-alkyle, hétérocyclyle et hétérocyclyl-alkyle ;

- $R_4$ représente une entité choisie parmi un atome d'hydrogène et les groupes alkyle en $C_{1-6}$, étant entendu que ces groupes alkyle peuvent ne porter aucun substituant ou porter 1 à 5 substituant(s) choisi(s) indépendamment parmi les substituants oxo, halogéno, cyano, nitro et azido, les substituants représentés par les formules $CF_3$, $CHF_2$, $CH_2F$, $OCF_3$, $OCHF_2$, $OCH_2F$, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''' et OR', et les substituants aryle, hétéroaryle, aryl-alkyle, hétéroaryl-alkyle, hétérocyclyle et hétérocyclyl-alkyle ;

- ou $R_3$ et $R_4$ peuvent représenter des entités qui forment, conjointement avec l'atome auquel elles sont liées, un groupe hétéroaryle ou hétérocyclique de 4 à 10 chaînons qui, dans chaque cas, ne porte aucun substituant ou porte 1 à 5 substituant(s) choisi(s) indépendamment parmi les substituants oxo, halogéno, cyano, nitro et azido, les substituants représentés par les formules $CF_3$, $CHF_2$, $CH_2F$, $OCF_3$, $OCHF_2$, $OCH_2F$, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''' et OR', et les substituants aryle, hétéroaryle, aryl-alkyle, hétéroaryl-alkyle, hétérocyclyle et hétérocyclyl-alkyle ;

- $R_5$ représente une entité choisie parmi un atome d'hydrogène et les groupes alkyle en $C_{1-6}$, étant entendu que ces groupes alkyle peuvent ne porter aucun substituant ou porter 1 à 5 substituant(s) choisi(s) indépendamment parmi les substituants oxo, halogéno, cyano, nitro et azido, les substituants représentés par les formules $CF_3$, $CHF_2$, $CH_2F$, $OCF_3$, $OCHF_2$, $OCH_2F$, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''' et OR', et les substituants aryle, hétéroaryle, aryl-alkyle, hétéroaryl-alkyle, hétérocyclyle et hétérocyclyl-alkyle ;

- ou $R_4$ et $R_5$ peuvent représenter des entités qui forment, conjointement avec l'atome auquel elles sont liées, un groupe carbocyclique, hétéroaryle ou hétérocyclique de 4 à 10 chaînons qui, dans chaque cas, ne porte aucun substituant ou porte 1 à 5 substituant(s) choisi(s) indépendamment parmi les substituants oxo, halogéno, cyano, nitro et azido, les substituants représentés par les formules $CF_3$, $CHF_2$, $CH_2F$, $OCF_3$, $OCHF_2$, $OCH_2F$, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''' et OR', et les substituants aryle, hétéroaryle, aryl-alkyle, hétéroaryl-alkyle, hétérocyclyle et hétérocyclyl-alkyle ;

- $R_6$ représente une entité choisie parmi les groupes trifluorométhyle, alkyle en $C_{1-10}$, cycloalkyle en $C_{3-10}$, aryle, aryl-alkyle, hétéroaryle, hétéroaryl-alkyle, hétérocyclyle et hétérocyclyl-alkyle, étant entendu que chacun de ces groupes alkyle, cycloalkyle, aryle, hétéroaryle et hétérocyclyle ne porte aucun substituant ou porte 1 à 5 substituant(s) choisi(s) indépendamment parmi les substituants oxo, halogéno, cyano, nitro et azido, les substituants représentés par les formules $CF_3$, $CHF_2$, $CH_2F$, $OCF_3$, $OCHF_2$, $OCH_2F$, NR'$SO_2$R'''', $SO_2$NR'R'', C(O)R', C(O)OR', OC(O)R', NR'C(O)OR'''', NR'C(O)R'', C(O)NR'R'', SR'''', S(O)R'''', $SO_2$R', NR'R'', NR'C(O)NR''R''', NR'C(NCN)NR''R''' et OR', et les substituants aryle, hétéroaryle, aryl-alkyle, hétéroaryl-alkyle, hétérocyclyle et hétérocyclyl-alkyle ;

- W représente une entité choisie parmi les groupes hétéroaryle comportant 1 à 4 hétéroatomes et les groupes hétérocyclyle comportant 1 à 4 hétéroatomes, étant entendu que chacun de ces groupes ne porte aucun substituant ou porte 1 à 5 substituant(s) symbolisé(s) par $ZR_{15}$, ou W représente un groupe symbolisé par $-C(O)OR_{15}$, $-C(O)NR_4R_{15}$, $-C(O)NR_4OR_{15}$, -C(O)-(cycloalkyle en $C_{3-10}$), -C(O)-(alkyle en $C_{2-10}$), - C(O)-(aryle), -C(O)-(hétéroaryle), -C(O)-(hétérocyclyle), $-S(O)_jNR_4R_{15}$, $-S(O)_jNR_4OR_{15}$, $-S(O)_jNR_4C(O)R_{15}$ ou $-C(O)NR_4S(O)_jR_6$,

où les symboles $R_4$ et $R_{15}$ ont les significations indiquées ici ou peuvent représenter des entités qui forment ensemble un cycle de 3 à 7 chaînons comportant 1 ou 2 atomes d'azote et, en option, un atome d'oxygène ;

- Z représente une liaison, un atome d'oxygène ou de soufre, ou un fragment symbolisé par $NR_{16}$ ou $NR_{16}SO_2$ ;
- $R_{15}$ représente une entité choisie indépendamment parmi un atome d'hydrogène, les groupes trifluorométhyle, alkyle en $C_{1-10}$, alcényle en $C_{2-10}$, alcynyle en $C_{2-10}$, cycloalkyle en $C_{3-10}$, (cycloalkyle en $C_{3-10}$)-alkyle, aryle, aryl-alkyle, hétéroaryle, hétéroaryl-alkyle, hétérocyclyle et hétérocyclyl-alkyle, étant entendu que chacun de ces groupes alkyle, alcényle, alcynyle, cycloalkyle, aryle, hétéroaryle et hétérocyclyle ne porte aucun substituant ou porte 1 à 5 substituant(s) choisi(s) parmi les substituants représentés par la formule $OR_3$ ou NR'R", où $R_3$ représente une entité choisie parmi un atome d'hydrogène et les groupes alkyle en $C_{1-4}$, alcényle en $C_{1-4}$ et (cycloalkyle en $C_{4-6}$)-alkyle, et où R' et R" représentent chacun, indépendamment, un atome d'hydrogène ou un groupe alkyle en $C_{1-4}$, ou R' et R" peuvent représenter des entités qui forment ensemble un cycle de 3 à 7 chaînons comportant 1 ou 2 atomes d'azote et, en option, un atome d'oxygène ;
- $R_{16}$ représente une entité choisie parmi un atome d'hydrogène et les groupes alkyle en $C_{1-10}$ ;
- ou $R_{15}$ et $R_{16}$ représentent des entités qui forment ensemble un cycle de 4 à 10 chaînons comportant 1 ou 2 atomes d'azote et, en option, un atome d'oxygène, lequel cycle ne porte aucun substituant ou porte 1 ou 2 substituant(s) alkyl-amino, amino, hydroxy ou alcoxy ;
- R' représente une entité choisie parmi un atome d'hydrogène et les groupes alkyle en $C_{1-4}$, alcényle en $C_{2-4}$, aryle et aryl-alkyle ;
- R" représente une entité choisie parmi un atome d'hydrogène et les groupes alkyle en $C_{1-4}$, alcényle en $C_{2-4}$, aryle et aryl-alkyle ;
- R"' représente une entité choisie parmi un atome d'hydrogène et les groupes alkyle en $C_{1-4}$, alcényle en $C_{2-4}$, aryle et aryl-alkyle ;
- R"" représente une entité choisie parmi les groupes alkyle en $C_{1-4}$, alcényle en $C_{1-4}$, aryle et aryl-alkyle ;
- ou deux quelconques des symboles R', R", R"' et R"" peuvent représenter des entités qui forment, conjointement avec l'atome auquel elles sont liées, un groupe carbocyclique, hétéroaryle ou hétérocyclique de 4 à 10 chaînons qui, dans chaque cas, peut en option porter 1 à 3 substituant(s) choisi(s) indépendamment parmi les substituants halogéno, cyano, nitro et azido, les substituants représentés par les formules $CF_3$, $CHF_2$, $CH_2F$, $OCF_3$, $OCHF_2$ et $OCH_2F$, et les substituants aryle, hétéroaryle, aryl-alkyle, hétéroaryl-alkyle, hétérocyclyle et hétérocyclyl-alkyle, et de préférence parmi les substituants halogéno, cyano, nitro, trifluorométhyle, difluorométhoxy, trifluorométhoxy et azido ;
- l'indice m vaut 0, 1, 2, 3, 4 ou 5 ;
- et l'indice j vaut 1 ou 2.

**2.** Composé de formule (I), conforme à la revendication 1, dans lequel :

- $R_1$, $R_2$, $R_9$ et $R_{11}$ représentent chacun, indépendamment, une entité choisie parmi les atomes d'hydrogène et d'halogène, les groupes alkyle en $C_{1-4}$, alcényle en $C_{1-4}$, alcynyle en $C_{2-4}$, cycloalkyle en $C_{3-4}$, cyano et nitro, et les groupes de formule $-OR_3$ ou $-NR_3R_4$, étant entendu que chacun de ces groupes alkyle, alcényle, alcynyle et cycloalkyle peut, en option, porter 1 à 5 substituant(s) halogéno ;
- $R_{10}$ représente une entité choisie parmi un atome d'hydrogène et les groupes de formule $-OR_3$, $-NR_4C(O)R_3$, $-C(O)NR_3R_4$ ou $-NR_3R_4$ ;
- L représente une liaison ou un groupe alkyle en $C_{1-5}$ ;
- $R_{12}$ représente une entité choisie indépendamment parmi les atomes d'hydrogène et d'halogène, les groupes alkyle en $C_{1-10}$, alcényle en $C_2$-10, alcynyle en $C_{2-10}$, cycloalkyle en $C_{3-10}$, cyano, nitro et azido, les groupes de formule $NR_4SO_2R_6$, $SO_2NR_3R_4$, $SO_2R_6$, $C(O)NR_3R_4$, $OR_3$, $NR_3R_4$, $-S(O)_jNR_4C(O)R_3$, $-C(O)NR_4S(O)_jR_6$, ou -S-(alkyle en $C_{1-2}$) portant 1 à 5 substituant(s) fluoro, étant entendu que chacun de ces groupes alkyle, alcényle, alcynyle, cycloalkyle, aryle, hétéroaryle et hétérocy-clyle ne porte aucun substituant ou porte 1 à 5 substituant(s) choisi(s) indépendamment parmi les substituants oxo, halogéno, cyano, nitro et azido, les substituants représentés par les formules $CF_3$, $CHF_2$, $CH_2F$, $OCF_3$, $OCHF_2$, $OCH_2F$, $SCF_3$, $SCHF_2$, $SCH_2F$, $NR_4SO_2R_6$, $SO_2NR_3R_4$, $C(O)R_3$, $C(O)OR_3$, $OC(O)R_3$, $NR_4C(O)OR_6$, $NR_4C(O)R_3$, $C(O)NR_3R_4$, $NR_3R_4$, $NR_5C(O)NR_3R_4$, $NR_5C(NCN)NR_3R_4$ et $OR_3$, et les substituants aryle, hétéroaryle, aryl-alkyle, hétéroaryl-alkyle, hétérocyclyle et hétérocyclyl-alkyle ;
- $R_{13}$ et $R_{14}$ représentent chacun, indépendamment, une entité choisie parmi les atomes d'hydrogène, de fluor et de chlore et les groupes alkyle en $C_{1-4}$, alcényle en $C_{2-4}$, alcynyle en $C_{2-4}$ et cycloalkyle en $C_{3-4}$, étant entendu que chacun de ces groupes alkyle, alcényle, alcynyle et cycloalkyle peut, en option, porter 1 à 5 substituant(s) halogéno ;
- W représente une entité choisie parmi les groupes hétéroaryle comportant 1 à 4 hétéroatomes et les groupes

hétérocyclyle comportant 1 à 4 hétéroatomes, étant entendu que chacun de ces groupes ne porte aucun substituant ou porte 1 à 3 substituant (s) symbolisé (s) par $ZR_{15}$,

ou W représente un groupe symbolisé par $-C(O)OR_{15}$, $-C(O)NR_4R_{15}$, $-C(O)NR_4OR_{15}$, -C(O)-(cycloalkyle en $C_{3-10}$), -C(O)-(alkyle en $C_{2-10}$), $-S(O)_jNR_4C(O)R_{15}$, $-C(O)NR_4S(O)_jR_6$, $-S(O)_jNR_4R_{15}$ ou $-S(O)_jNR_4OR_{15}$ ;

- Z représente un atome d'oxygène ou un fragment symbolisé par $NR_{16}$ ou $NR_{16}SO_2$ ;
- $R_{15}$ représente une entité choisie parmi un atome d'hydrogène et les groupes alkyle en $C_{1-4}$, alcényle en $C_{2-4}$ et (cycloalkyle en $C_{4-6}$)-alkyle, étant entendu que chacun de ces groupes alkyle et alcényle peut en outre porter 1 ou 2 substituant(s) hydroxy, alcoxy en $C_{1-4}$ ou de formule NR'R" ;
- $R_{16}$ représente une entité choisie parmi un atome d'hydrogène et les groupes alkyle en $C_{1-4}$ ;
- et R' et R" représentent chacun, indépendamment, une entité choisie parmi un atome d'hydrogène et les groupes alkyle en $C_{1-4}$, alcényle en $C_{2-4}$, aryle et aryl-alkyle.

**3.** Composé de formule (I), conforme à la revendication 1 ou 2, dans lequel:

- $R_1$ représente une entité choisie indépendamment parmi les atomes d'hydrogène et de fluor ;
- $R_1$ représente une entité choisie indépendamment parmi les atomes de fluor et de chlore et le groupe méthyle, lequel groupe méthyle peut, en option, porter 1 à 3 substituant(s) fluoro ;
- $R_9$ représente une entité choisie indépendamment parmi les atomes d'hydrogène, de fluor et de chlore ;
- $R_{10}$ représente une entité choisie parmi un atome d'hydrogène et les groupes de formule $-OR_3$ ou $-NR_3R_4$ où $R_3$ et $R_4$ représentent chacun, indépendamment, un groupe alkyle en $C_{1-6}$ qui peut, en option, porter 1 ou 2 substituant(s) alkylamino ou alcoxy, ou $R_3$ et $R_4$ représentent des entités qui forment conjointement un groupe cyclique comportant 1 ou 2 atomes d'azote et, en option, un atome d'oxygène, lequel cycle peut, en option, porter 1 ou 2 substituant(s) alkylamino ou alcoxy ;
- L représente une entité choisie parmi une liaison et les groupes méthanediyle, éthanediyle, n-propanediyle et n-butanediyle ;
- $R_{11}$ représente une entité choisie indépendamment parmi les atomes d'hydrogène, de fluor, de chlore et de brome et les groupes méthyle et méthoxy, dont les groupes méthyle peuvent, en option, porter 1 à 3 substituant (s) fluoro ;
- $R_{12}$ représente une entité choisie indépendamment parmi les atomes d'hydrogène, de fluor, de chlore, de brome et d'iode, les groupes nitro, méthyle, éthyle, n-propyle, isopropyle, cyclopropyle et méthoxy, et les groupes de formule $SCF_3$, $SCHF_2$, $SCH_2F$, $SO_2NR_3R_4$ ou $C(O)NR_3R_4$,

où les groupes méthyle peuvent, en option, porter 1 à 3 substituant(s) fluoro et où $R_3$ et $R_4$ représentent chacun, indépendamment, un groupe alkyle en $C_{1-6}$ qui peut, en option, porter 1 ou 2 substituant(s) alkylamino ou alcoxy, ou $R_3$ et $R_4$ représentent des entités qui forment conjointement un groupe cyclique comportant 1 ou 2 atomes d'azote et, en option, un atome d'oxygène, lequel cycle peut, en option, porter 1 ou 2 substituant(s) alkylamino ou alcoxy ;

- $R_{13}$ représente une entité choisie indépendamment parmi les atomes d'hydrogène et de fluor ;
- $R_{14}$ représente une entité choisie indépendamment parmi les atomes d'hydrogène et de fluor ;
- et W représente un groupe symbolisé par $C(O)NR_4OR_{15}$ ou $SO_2NR_4OR_{15}$,

ou W représente un fragment de formule

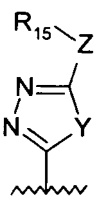

dans laquelle
Z représente un groupe symbolisé par $NR_{16}$,

$R_{15}$ représente un groupe alkyle en $C_{1-4}$ ou alcényle en $C_{1-4}$, qui peut, en option, porter 1 à 3 substituant(s) hydroxy, méthoxy, amino, diméthylamino, méthyl-amino, éthyl-amino ou diéthyl-amino,
$R_{16}$ représente un atome d'hydrogène ou un groupe alkyle en $C_{1-4}$, ou $R_{15}$ et $R_{16}$ représentent des entités qui forment conjointement un groupe cyclique de 4 à 10 chaînons, comportant 1 ou 2 atomes d'azote et, en option, un atome d'oxygène, lequel cycle peut, en option, porter 1 ou 2 substituant(s) alkylamino, amino, hydroxy ou alcoxy, et Y représente un chaînon symbolisé par O, S ou NR'.

**4.** Composé de formule (1), conforme à l'une des revendications 1 à 3, dans lequel :

W représente un groupe symbolisé par $C(O)NR_4OR_{15}$ ou $SO_2NR_4OR_{15}$,

ou W représente un fragment de formule

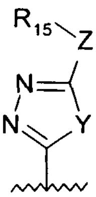

,

où $R_4$ représente un atome d'hydrogène,

Z représente un groupe symbolisé par NH,
$R_{15}$ représente un groupe alkyle en $C_{1-4}$ ou alcényle en $C_{1-4}$, qui peut, en option, porter 1 ou 2 substituant(s) hydroxy, alcoxy en $C_{1-4}$ ou de formule NR'R",
R' et R" représentent chacun, indépendamment, un atome d'hydrogène ou un groupe méthyle ou éthyle,
et Y représente un chaînon symbolisé par O.

**5.** Composé de formule (I), conforme à l'une des revendications 1 à 4, dans lequel les symboles L-$R_{10}$ représentent conjointement un groupe méthyle ou un atome d'hydrogène.

**6.** Composé conforme à l'une des revendications 1 à 5, pour son utilisation en tant que médicament.

**7.** Composé conforme à l'une des revendications 1 à 5, pour son utilisation dans le traitement de maladies ou d'affections hyperprolifératives médiées par une prolifération aberrante, y compris un cancer.

**8.** Composé conforme à l'une des revendications 1 à 5, pour son utilisation dans le traitement de maladies hyperprolifératives en rapport avec une hyperactivité de MEK, ainsi que de maladies modulées par la cascade MEK, chez des mammifères.

**9.** Composé conforme à la revendication 8, conçu pour le traitement de maladies choisies dans l'ensemble formé par les cancers, inflammations, pancréatite, maladies des reins, douleurs, hyperplasie bénigne de la peau, resténose, adénome de la prostate, maladies en rapport avec l'angiogenèse ou la vasculogenèse, angiogenèse de tumeur, maladies de peau choisies parmi psoriasis, eczéma et sclérodermie, diabète, rétinopathie diabétique, rétinopathie du prématuré, dégénérescence maculaire liée à l'âge, hémangiome, gliome, mélanome et sarcome de Kaposi.

**10.** Composé conforme à la revendication 8 ou 9, conçu pour le traitement d'un cancer ou d'une inflammation.

**11.** Composé conforme à l'une des revendications 8 à 10, conçu pour le traitement d'un cancer, choisi dans l'ensemble formé par les cancers des ovaires, du sein, du poumon, du pancréas, de la prostate ou du côlon et le carcinome épidermoïde, ou d'une inflammation choisie dans l'ensemble formé par la polyarthrite rhumatoïde, les affections intestinales inflammatoires et l'athérosclérose.

**12.** Utilisation d'un composé conforme à l'une des revendications 1 à 5, dans la préparation d'un médicament destiné au traitement de maladies ou d'affections hyperprolifératives médiées par une prolifération aberrante, y compris un cancer, et de maladies hyperprolifératives en rapport avec une hyperactivité de MEK, ainsi que de maladies modulées par la cascade MEK, chez des mammifères.

13. Composition pharmaceutique comprenant un composé conforme à l'une des revendications 1 à 5 et un véhicule pharmacologiquement admissible.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5525625 A
- WO 9843960 A
- WO 9901421 A
- WO 9901426 A
- WO 0041505 A
- WO 0042002 A
- WO 0042003 A
- WO 0041994 A
- WO 0042022 A
- WO 0042029 A
- WO 0068201 A
- WO 0168619 A
- WO 0206213 A
- WO 03035626 A2
- WO 03077855 A
- WO 03077914 A
- WO 2004005284 A
- WO 2004056789 A
- WO 0206213 A2
- WO 0105391 A


### Non-patent literature cited in the description


- **Steelman et al.** *Leukemia,* 2004, vol. 18, 189-218
- **Stirewalt et al.** *Blood,* 2001, vol. 97, 3589-95
- **Kohl et al.** *Science,* 1993, vol. 260, 1834-1837
- **McCubrey et al.** *Int J Oncol,* 1995, vol. 7, 295-310
- **Davies, H et al.** *Nature,* 2002, vol. 417, 949-954
- **Chang et al.** *Leukemia,* 2003, vol. 17, 1263-93
- **Lewis et al.** *Adv. Cancer Res,* 1998, vol. 74, 49-139
- **Crews et al.** *Science,* 1992, vol. 258, 478-80
- **Huang et al.** *Mol Biol Cell,* 1995, vol. 6, 237-45
- **Lin et al.** *Genes Dev,* 1998, vol. 12, 3008-3019
- **Carter et al.** *J Biol Chem,* 2000, vol. 275, 27858-64
- **Lee et al.** *Nature,* 1994, vol. 372, 739-746
- **Dudley et al.** *Proc. Natl. Acad. Sci. U.S.A.,* 1995, vol. 92, 7686-7689
- **Yeh, T. et al.** *Proceedings of the American Association of Cancer Research,* 2004, 45
- **Lee, P. et al.** *Proceedings of the American Association of Cancer Research,* 2004, 45
- **Wallace, E. et al.** *Proceedings of the American Association of Cancer Research,* 2004, 45
- **Swanton C ; Johnston S.** *IDDB MEETING REPORT,* February 2003
- **Waterhouse et al.** *Proceedings of the American Society for Clinical Oncology,* 2003, 22
- March's Advanced Organic Chemistry: Reactions, Mechanisms, and Structure. John Wiley & Sons
- **DenHertog.** *Recl Trav Chim Pays-Bas,* 1946, vol. 65, 129-140
- **Theophil Eicher ; Siegfried Hauptmann.** The Chemistry of Heterocycles; Structures, Reactions, Synthesis and Application. Wiley-VCH, 2003